(19) Europäisches Patentamt
European Patent Office
Office européen des brevets

(11) EP 4 684 779 A1

(12) **EUROPEAN PATENT APPLICATION**

(43) Date of publication:
**28.01.2026 Bulletin 2026/05**

(21) Application number: **24382805.0**

(22) Date of filing: **23.07.2024**

(51) International Patent Classification (IPC):
***A61K 9/51*** *(2006.01)* ***A61K 9/00*** *(2006.01)*
***A61K 47/22*** *(2006.01)*

(52) Cooperative Patent Classification (CPC):
**A61K 9/5123; A61K 9/0019; A61K 31/7105;**
A61K 47/22

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Diversa Technologies S.L.**
**15782 Santiago de Compostela (ES)**

(72) Inventors:
- **DE LA FUENTE FREIRE, María**
  **15782 Santiago de Compostela (ES)**
- **TAINA GONZÁLEZ, Laura**
  **15782 Santiago de Compostela (ES)**
- **ABAL SANISIDRO, Marcelina**
  **15782 Santiago de Compostela (ES)**
- **VÁZQUEZ RÍOS, Abi Judit**
  **15782 Santiago de Compostela (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **LIPID NANOSYSTEMS**

(57) The present invention relates to a lipid system comprising:
a) at least one oil,
b) at least one ionizable lipid,
c) at least one sphingolipid, and
d) at least one lipid-polymer,
with the proviso is that the lipid system does not comprise cholesterol.

The present invention also uses of the lipid system in therapy.

EP 4 684 779 A1

## Description

### TECHNICAL FIELD

The present invention relates to the field of nanotechnology. Particularly, the present invention relates to the field of lipid nanosystems, preferably sphingolipid nanosystems.

### BACKGROUND ART

Gene therapy opens promising venues for developing innovative treatments against cancer, immunotherapies or rare diseases. In this regard, mRNA-based therapies hold important advantages such as the increased delivery efficiency, as mRNA does not need to reach the nucleus for effective protein translation, which in turn allows for higher safety profiles. Furthermore, the ease in production and mRNA sequence modification makes it a scalable technology, including for GMP manufacturing. Promising therapies are based on small interfering RNA (siRNA), Micro RNA (miRNA), short activating RNA (saRNA), antisense oligonucleotides (ASOs), complementary DNA (cDNA), complementary RNA (cRNA), long non-coding RNA (IncRNA), Aptamers, plasmid DNA (pDNA), deoxyribonucleic acid (DNA), delf-amplifying DNA (saDNA), messenger RNA (mRNA), single guide RNA (sgRNA), among others. CRISPR/Cas9 microbial genome-editing technology for example enables permanent repair of genetic mutations that cause certain diseases.

The field of gene therapy requires safe and efficient methods for delivering nucleic acids (DNA and RNA) to target cells. While viral vectors based on adenovirus or adeno-associated virus (AAVs) have been traditionally used due to high editing efficiency, they carry significant safety risks. These limitations necessitate the development of innovative, non-viral delivery systems. In this regard, a promising alternative are non-viral nanoparticles, such as polymeric nanoparticles or lipid-based nanoparticles. Specifically, for efficient RNA delivery, the field of nanomedicine has made significant advances for various applications, for example with the approval of Onpattro in 2018 [8], COVID-19 vaccines in 2020 [9.10] , and RSV Vaccine in 2024. Notably, these breakthroughs rely on lipid-based particles as the RNA delivery technology of choice, representing the most advanced non-viral vector for RNA delivery to date. In fact, various technologies using lipids have been proposed for the development of RNA nanomedicines, including lipoplexes and cationic lipid nanoemulsions [12]. Gene editing has also been explored using lipid-based systems. Thanks to nanoparticles' ability to encapsulate and/or associate large components such as long RNAs and large proteins, their use for CRISPR technology (i.e. delivery and co-delivery of ribonucleoprotein (RNP) complexes and Cas9 DNAs or RNAs) has been previously investigated.

In the development of innovative delivery systems for gene therapy and gene editing applications there are some key elements to consider. Critical among these is versatility. Innovative nanosystems should be rapidly adaptable to deliver different types of nucleic acids, including DNA, mRNA, siRNA, and others, both individually and in combination with each other or other molecules. This versatility allows for the development of targeted therapies tailored to specific genetic conditions, aligning well with advancements in personalized medicine.

Another key element is modularity. The biodistribution of these nanoparticles should be easily tailored through simple modifications to target specific tissues, organs, cells, and even cellular compartments. This modularity makes them suitable for a wide range of therapeutic applications.

Finally, simplicity is crucial. Innovative nanosystems should be designed using minimal components while still achieving optimal properties for delivery. This focus on simplicity translates to easier manufacturing processes and potentially lower costs.

Therefore, there is a need to develop improved nanosystems.

### DESCRIPTION OF DRAWINGS

**Figure 1. FLuc-mRNA SNPs containing ionizable lipids overperform SNPs containing cationic lipids.** A) Colloidal properties (size and PDI) and B) Surface Charge (mV) of FLuc-SNPs containing C12-200 (C12) or 306-O12B (306) ionizable lipids and DOTAP, DOTMA and DDA cationic lipids. C) Transfection efficiency with FLuc-mRNA loaded SNPs represented as the Mean and SD of bioluminescence (Arbitrary Units. A.U.) catalyzed by the *in vitro* translated protein in cells transfected with SNPs. Transfections were performed with 100ng of mRNA at a final concentration of 1μg/mL.

**Figure 2. Colloidal Properties of FLuc-mRNA SNPs containing variable amounts of VE and SM (namely VEx2, VE, SM, SMx2 SNPs) and prepared with the ionizable lipid C12-200. A)** Size distribution (nm) and concentration (particles/mL) measured by NTA. **B)** Transmission Electron Microscopy (TEM) images of SNPs upon sample preparation and staining with 2% Phosphotungstic Acid. Pictures were acquired at 10.000X for VEx2-SNPs.

20.000X VE-SNPs and SM-SNPs and 50.000X for SMx2-SNPs. **C)** Colloidal Stability (mean size and polydispersity index, PDI) of SNPs stored at 4°C for up to 7 days. **D)** Cell uptake measured as the percentage (%) of positive HEPG2 cells that efficiently internalized Top-Fluor-labelled SNPs determined by flow cytometry. **E)** Cell uptake in HEK293 cells measured as mean intensity for Cy5-mRNA loaded SM-SNPs, after 2h incubation. The percentage of positive events is 93.7 ±0.3. **F)** Transfection efficiency in HEK293 cells transfected with eGFP-mRNA loaded SNP determined by flow cytometry after 24h incubation. The percentage of positive events is 99.1 % ±0.4. **G)** Transfection efficiency with FLuc-mRNA loaded SNPs represented as the Mean and SD of bioluminescence (Arbitrary Units. A.U.) catalyzed by the *in vitro* translated protein in cells transfected with SNPs that associate mRNA-Luc. Onpattro® composition (ONP) and commercial transfection reagent MessengerMax (C) was used as positive control. Transfections were performed with 100ng of mRNA, at a final concentration of 1μg/mL. Statistical analysis performed with One-way ANOVA ± SD of Log (Y) and Two-way ANOVA ± SD of Log (Y) and was followed by Tukey's multiple comparison test. (ns=0.1234; ****p<0.0001; ***p=0.0002; **P=0.0021; *p=0.033).

**Figure 3. Toxicity Assessment of SNPs (SM/VE/SMG-PEG) with variable content in VE and SM (namely VEx2, VE, SM, SMx2) and prepared with the ionizable lipid C12-200.** Cell viability in A) HEK293 and B) HEPG2 determined using a Cell-Titer® fluorescence kit (Promega) after 24h incubation with FLuc-mRNA loaded SNPs (cells incubated with 100ng of mRNA at a final concentration of 1μg/mL). C) Images of HEPG2 cells incubated with FLuc-mRNA loaded SNPs during 48h, or with the control reagent MessengerMAX. Images Acquired at 10X in an optical microscope

**Figure 4. Characterization of SNPs (SM/VE/DMG-PEG) prepared with different types of ionizable lipids. A)** Mean size and polydispersity index (Pdl) of FLuc-mRNA loaded SNPs prepared with MC3, C12-200, DLin-DMA (DLin), 306-O12B (306) and DLin-KC2-DMA (KC2) ionizable lipids. **B)** Transfection efficiency in HEK293 cells transfected with FLuc-mRNA loaded SNPs prepared with MC3 and C12-200 ionizable lipids measured as the bioluminescence produced by the protein (in A.U).Cells were incubated with 100ng of mRNA at a final concentration of 1μg/mL.

**Figure 5. Characterization of SNPs prepared with different types of helper lipid and lipid-polymers. A)** Size and Pdl of SNPs (C12/SM/VE/DMG-PEG:helper) prepared with the ionizable lipid C12-200 and helper lipids DOTAP or DOPE. **B)** Size and Pdl of SNPs (ionizable/SM/VE/lipid-polymer) prepared with the ionizable lipids C12-200 or 306-O-12B with or without the helper lipid DOPE and with the lipid-polymers DMPE-Psar and VE-Psar at two different percentages , 1% and 3%. **C)** Transfection efficiency in HEK293 cells with FLuc-mRNA loaded SNPs D) Transfection efficiency in mice with FLuc-mRNA SNPs (namely SMx2) and formulations with helper and different Lipid-Polymer compounds. Bioluminescence (ph/s/cm$^2$/sr) acquired after 6h of intravenous administration (tail injection, 10 μg of mRNA).

**Figure 6. Modulation of biodistribution as a matter of composition. A)** Biodistribution assays were carried out with FLuc-mRNA loaded SNPs prepared with the ionizable lipids C12-200 (C12) or 306-O12-B (306) with or without the addition of DOPE (namely C12SMx2 (C12/SM/VE/DMG-PEG and 306-SMx2 DOPE or Biodegradable DOPE (306/SM/VE/DMPE-Psar, respectively). Transfection efficiency in mice determined at 6h or 24h after intravenous administration (tail injection. 10 μg of mRNA). Bioluminescence (ph/s/cm$^2$/sr). ROI (Total Flux (p/s)) used for quantification. **B)** Organ biodistribution (Liver, spleen, heart, kidneys, and lung). calculated as a percentage (%) of total bioluminescence (ph/s/cm$^2$/sr) after 24h post-administration and organ dissection.

**Figure 7. Characterization and transfection efficiency of SNPs (C12/SM/Oil/DMG-PEG) prepared with different oils and the ionizable lipid C12-200, namely Vitamin E (VE), Oleic acid and Miglyol.** A) Size and polydispersity Index (Pdl) of FLuc-mRNA loaded SNPs containing Vitamin E, Oleic Acid or Miglyol. B) Transfection efficiency in HEK293 cells determined by the expression of the encoded bioluminescent protein luciferase, expressed in Arbitrary Units (A.U.) (1 g/mL final dose of mRNA-Luc. analysed 24h post-transfection).

**Figure 8. Characterization of SNPs (C12/SM type/VE/DMG-PEG) prepared with Vitamin E and C12-200, and different types of sphingolipids (Natural SM or SM, SM2:02, SM 18:1, SM 24:1, SM 24, Sphyngosine SM 16:1).** Average size and polydispersity index (Pdl).

**Figure 9. Characterization of SNPs (C12/SM/VE/DMG-PEG) prepared with high percentages (80% or higher) of one of the main three components, 80% ionizable lipid (C12), 90% Vitamin E or 90% sphingomyelin.** Size and polydispersity index (Pdl).

**Figure 10. Characterization of SNPs (ionizable/SM/VE/helper/DMG/PEG-Mal or PEG-DBCO) prepared with lipid-PEG derivatives that contain the reactive groups DBCO and Maleimide and different ionizable and helper lipids. A)** Size and polydispersity index (Pdl) of formulations prepared with lipid-PEG-DBCO (DBCO) lipid-PEG-Maleimide (MALEIMIDE).

**Figure 11. Characterization of SNPs (C12/SM/VE/DMG-PEG) that encapsulate different types of nucleic acids (A) and combinations.** Size and polydispersity index (Pdl) of formulations that encapsulate Aptamer, mRNA, pDNA and siRNA.

## GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

If the term "about" as used in connection with a numerical value throughout the specification and the claims denotes an interval of accuracy familiar and acceptable to a person skilled in the art. For instance, the term "about" means the indicated value $\pm$ 1% of its value, or the term "about" means the indicated value $\pm$ 2% of its value, or the term "about" means the indicated value $\pm$ 5% of its value, the term "about" means the indicated value $\pm$ 10% of its value, or the term "about" means the indicated value $\pm$ 20% of its value, or the term "about" means the indicated value $\pm$ 30% of its value; preferably the term "about" means exactly the indicated value ($\pm$ 0%).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise" and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The use of any and all examples, or exemplary language (e.g. "such as") provided herein, is intended merely to better illuminate the invention and does not pose a limitation on the scope of the invention unless otherwise claimed. No language in the specification should be construed as indicating any non-claimed element as essential to the practice of the invention.

## DESCRIPTION OF THE EMBODIMENTS

Various technologies have been proposed for the development of RNA nanomedicines, including lipidic vehicles, lipoplexes and cationic lipid nanoemulsions. However, they present associated high immunogenicity and toxicity, hindering clinical translation. Thus, new delivery methods based on the use of nanotechnology are needed.

In this study, we have developed sphingomyelin nanoparticles (SNPs) for the efficient delivery of mRNA and other nucleic acids, alone and in combination, including combination with proteins and polymers, as shown in the Examples.

Example 1 shows the importance of including an ionizable lipid rather than a cationic lipid.

The inventors aimed at formulating 4-component SNPs for mRNA delivery, containing as main components Vitamin E (VE) and sphingomyelin (SM), in combination with an ionizable lipid and a lipid-polymer derivative, see the examples.

The Examples contain information related to stability and functionality, and ability to modulate the *in vitro* and *in vivo* behavior by modifying the ratio of VE and SM. The influence to include helper lipids in the formulations is also

showcased.

The Examples also show that SNPs can be easily modified with a broad range of materials, allowing for different applications and properties such as increased potency, complete biodegradability. As well, authors highlight the incorporation of oils different to VE, such as Oleic Acid and Miglyol. In addition, the inventors highlight the possibility to use different sphingolipids to formulate such SNPs as well as the maximization of ionizable lipid, VE, or SM within the formulation without altering stability, showcasing once more the broad usage.

The Examples also show the possibility of modulating SNPs for the use of different nucleic acids such as siRNA, aptamers, pDNA in combination with other elements such as proteins.

Overall, the present invention shows the efficacy of using cholesterol-free lipidic systems containing at least one oil, one ionizable lipid, one sphingolipid, and one lipid-polymer derivative as vehicles to deliver therapeutic agents to a target cell or tissue.

In sum, the present invention describes a novel non-viral nanoparticle platform. By incorporating unique features, this platform enhances delivery efficiency, biocompatibility, and targeting capabilities. The following sections will detail the composition, design, and functionalities of this innovative platform, highlighting its potential to revolutionize nucleic acid delivery for gene therapy applications.

In **a first aspect,** the present invention provides a composition comprising a lipid system, also named herein the "lipid system of the invention", wherein the lipid system is characterized by comprising:

a. At least one oil,
b. At least one ionizable lipid,
c. At least one sphingolipid,
d. At least one lipid-polymer or lipid-polymer derivative,
e. Optionally, at least a helper lipid, and
f. Optionally, a surface-decoration moiety.

In a prefer embodiment, the lipid system is a lipid nanosystem or lipid nanoparticle, also named herein SNPs. As used herein "lipid nanosystems" or "lipid nanoparticles" or "sphingolipid nanoparticles" or "SNPs" are understood as particles whose main components are lipids and have a mean size in the nanometer ranges. Preferably, said lipid nanoparticle has an average diameter of below 1000 nm, preferably between 10 and 500 nm, most preferably between 30-300 nm. By "average diameter" is meant the hydrodynamic diameter of the particles that diffuse at the same speed. This average particle size is preferably measured using the Dynamic Light Scattering (DLS) technique.

In an embodiment, the lipid system may comprise two, three, four, or more populations of lipid nanoparticles or SNPs. By "population of lipid nanoparticles" is meant a plurality SNPs that are homogeneous in their sizes and shapes, and that form a colloidal dispersion. By "homogeneous" is meant that the particles in each of the populations of SNPs according to the first aspect are characterized by having substantially the same particle size or diameter, that is, for being substantially monodisperse.

In an embodiment, the plurality of SNPs in a population of SNPs are at least 70%, 80%, 90%, 95%, 96%, 97%, 98%, 99%, or 100% identical among them in a population of SNPs. By "identical" is meant that the SNPs are substantially similar in their characteristics. including shape, composition, properties, coating, and functionalization.

Preferably, the lipid system of the invention does not comprise:

- cholesterol,
- DSPC, or
- DSPC and cholesterol.

Each of the components of the lipid system of the invention are described in detail below:

a) <u>The at least one oil.</u>

As explained above, the lipid system, preferably lipid nanosystem or lipid nanoparticle or population of lipid nanoparticles, comprises one or more oils or oils derivatives.

As used herein "oil" shall be understood as a viscous, high density, liquid substance that is generally unctuous. It is a hydrophobic liquid, meaning it is not miscible with water, and can be derived from various sources, and they are composed of different types of molecules.

Preferably, said oils are selected from the list consisting of $\alpha$ tocopherol (vitamin E), Miglyol, olive (oleic acid), sunflower oil, peanut, avocado, argan, almond, calendula, coconut, wheat germ, arnica, borage, sesame, cotton, castor bean, safflower, palm, wheat germ, tea tree, jojoba, linseed, silicone, glycerol, triglyceride oils, hypericum, rosehip, isopropyl myristate, squalene, tributyrin, or any combination thereof.

More preferably, the oil is selected from the group of Vitamin E, olive oil, and Miglyol. Most preferably, the oil is Vitamin E (also referred herein to as "VE").

Vitamin E is a collective term for several biologically similar compounds, including those called tocopherols and tocotrienols, which share the same biological activity. Vitamin E acts as a scavenger of free radicals. In addition, Vitamin E helps to block lipid peroxidation and may also form an important part of the membrane structure due to its interaction with membrane phospholipids. Forms of Vitamin E for the present invention include Alpha-tocopherol, D-alpha-tocopherol, D-alpha-tocopherol acetate, DL-alpha-tocopherol and DL-alpha-tocopherol acetate or any derivatives thereof.

b) The at least one ionizable lipid.

As explained above, the lipid system, preferably lipid nanosystem or lipid nanoparticle or population of lipid nanoparticles, comprises one or more ionizable lipids.

As used herein "ionizable lipid" is understood as a lipid molecule with one, two or several primary, secondary, tertiary amines which are ionized and protonated at a pH below their pKa, and thus are capable of electrostatically bind to nucleic acids, thus, allowing for nucleic acid association.

The ionizable lipids can be classified depending on their structure into i) Unsaturated, ii) Branched or multi-tail, and iii) Biodegradable.

1. Unsaturated ionizable lipids are lipids containing at least one primary, secondary, or tertiary amine with at least one tail. These tail or tails, in turn, present unsaturations (*i.e.,* presence of double bonds within the alkyl chain), an aspect that has been associated with increased membrane fluidity and thus, increased endosomal escape. Preferred lipids of this category that may be present in the lipid system of the invention are:

    a. DODMA, (N,N-dimethyl-2,3-*bis*[(9Z)-9-octadecen-1-yloxy]-1-propanamine),
    b. DLin-DMA lipid, also called DLin, (N,N-dimethyl-2,3-bis[(9Z,12Z)-9,12-octadecadien-1-yloxy]-1-propanamine),
    c. DLin-KC2-DMA, also called KC2, (N,N-dimethyl-2,2-di-(9Z,12Z)-9,12-octadecadien-1-yl-1,3-dioxolane-4-ethanamine), and
    d. DLin-MC3-DMA lipid, also called MC3, (4-(dimethylamino)-butanoic acid, (10Z, 13Z)-1 -(9Z, 12Z)-9,12-octadecadien-1-yl-10,13-nonadecadien-1-yl ester).

2. Multi-tail ionizable lipids are lipids containing at least one primary, secondary, or tertiary amine and, in addition, at least three tails which in turn may or may not present tail branches which may or may not present unsaturation (i,e., double bonds). These lipids include materials with a lipid-polymeric nature as well. Preferred lipids of this category that may be present in the lipid system of the invention are:

    a. SM-102, also called Lipid H, (8-[(2-hydroxyethyl)[6-oxo-6-(undecyloxy)hexyl]amino]-octanoic acid, 1-octylnonyl ester),
    b. ALC-0315 (2-hexyl-decanoic acid, 1,1'-[[(4-hydroxybutyl)imino]di-6,1-hexanediyl] ester),
    c. C12-200 (1,1'-[[2-[4-[2-[[2-[*bis*(2-hydroxydodecyl)amino]ethyl](2-hydroxydodecyl)amino]ethyl]-1-piperazinyl]ethyl]imino]*bis*-2-dodecanol),
    d. CKK-E12 (3,6-*bis*[4-[*bis*(2-hydroxydodecyl)amino]butyl]-2,5-piperazinedione),
    e. 306-Oi10 (tetrakis(8-methylnonyl) 3,3',3",3‴-(((methylazanediyl)*bis*(propane-3,1-diyl))bis(azanetriyl))tetrapropionate), and
    f. 9A1P9 (2-(dioctylamino)ethyl nonyl hydrogen phosphate).

3. Biodegradable ionizable lipids are lipids containing at least one primary, secondary, or tertiary amine which may or may not present two or more tails and, in turn, may or may not present unsaturations (i.e. double bonds within their alkyl chains) but, whose chemical entities (for instance, but not only, presence of ester bonds), allow for complete metabolization (i.e. degradation to available compounds) within the body. Preferred lipids of this category that may be present in the lipid system of the invention are:

    a. 306-O12B, also referred to as 306, tetrakis(2-(octyldisulfaneyl)ethyl) 3,3',3",3‴-(((methylazanediyl)bis(propane-3,1 diyl))bis(azanetriyl)) tetrapropionate
    b. OF-Deg-Lin, 9Z.12Z-octadecadienoic acid, 9Z,12Z-octadecadienoic acid, 1,1,1",1‴-[(3,6-dioxo-2,5-piperazinediyl)bis(4,1-butanediylnitrilodi-2,1-ethanediyl)] ester, and
    c. L319, 9-[4-(dimethylamino)-1-oxobutoxy]-heptadecanedioic acid, 1,17-di-(2Z)-2-nonen-1-yl ester.

Preferably, the ionizable lipid is C12-200 (1,1'-[[2-[4-[2-[[2-[bis(2-hydroxydodecyl)amino]ethyl](2-hydroxydodecyl)amino]ethyl]-1-piperazinyl]ethyl]imino]bis-2-dodecanol) or 306-O12B , tetrakis(2-(octyldisulfaneyl)ethyl) 3,3',3",3'''-(((methylazanediyl)bis(propane-3,1 diyl))bis(azanetriyl)) tetrapropionate.

c) The at least one sphingolipid

As explained above, the lipid system, preferably lipid nanosystem or lipid nanoparticle or population of lipid nanoparticles, comprises one or more sphingolipids.

As used herein "sphingolipid" shall be understood as a class of lipids containing a sphingoid base backbone (long-chain amino alcohol that serves as a key structural component). They are generally classified into phosphosphingolipids and glycosphingolipids.

Preferably, the sphingolipid present in the lipid system of the invention is selected from the list consisting of:

- Sphingomyelin ((N-hexadecanoyl-D-erythro-sphingosylphosphorylcholine N-(hexadecanoyl)-sphing-4-enine-1-phosphocholine),
- sphingosine,
- phosphosphyngolipids,
- glycosphingolipid,
- ceramide (preferably used as a stabilizer or surfactant), or
- any derivative thereof.

As used herein "Phosphosphingolipids" refers to a group of sphingolipids that have been phosphorylated at the C-1 hydroxyl group of the sphingoid base. The sphingoid base can also be linked to a fatty acid through an amide bond as well as the phosphate group can be attached to various groups like choline or ethanolamine. The phosphosphingolipids with unmodified phosphate groups are involved in many signaling pathways. The sphingolipids with modified phosphate groups play structural roles in biological membranes.

As used herein "glycosphingolipid" refers to lipids that have glycans attached to the C-3 hydroxyl group of diacylglycerol. Sugars that are linked to glycosphingolipids are wide in variety.

In the present invention, "sphingomyelin", also called herein "SM", is understood to mean a lipid of the sphingolipid family, being the most common, found in cell membranes, having the following chemical structure:

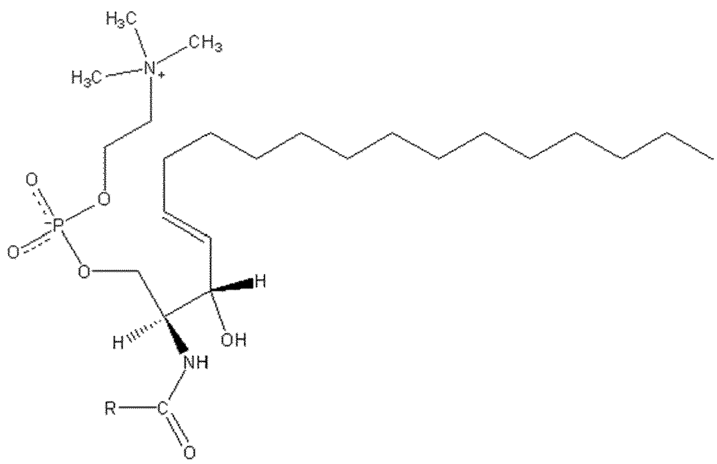

Preferably, the sphingolipid is selected from the list consisting of:

- SM (N-hexadecanoyl-D-erythro-sphingosylphosphorylcholine N-(hexadecanoyl)-sphing-4-enine-1-phosphocholine),
- SM 24:1 Nervonoyl Sphingomyelin N-(15Z-tetracosenoyl)-sphing-4-enine-1-phosphocholine,
- SM 18:1 N-(9Z-octadecenoyl)-sphing-4-enine-1-phosphocholine,
- SM 16:1 N-palmitoleoyl-D-erythro-sphingosylphosphorylcholine,
- SM 2:0 N-acetyl-D-erythro-sphingosylphosphorylcholine, and
- SM 24 N-(tetracosanoyl)-sphing-4-enine-1-phosphocholine.

Preferably, the sphingolipid present in the lipid system of the present invention is sphingomyelin, or a derivative

thereof.

d) The at least one lipid-polymer or lipid-polymer derivative.

As explained above, the lipid system, preferably lipid nanosystem or lipid nanoparticle or population of lipid nanoparticles, comprises one or more lipid-polymer or lipid-polymer derivatives.

As used herein "lipid-polymer" or "lipid-polymer derivative" shall be understood as an amphiphilic molecule composed of a lipid of any nature or kind linked to a polymer of any nature, which plays an active role on stabilizing the nanosystems.

The lipid comprised in the lipid-polymer is preferably a phospholipid.

The polymer comprised in the lipid-polymer is preferably be composed of monomers of amino acid nature, *i,e,* polyaminoacids (such as sarcosine, or other), carbohydrate or saccharide nature, *i,e,* polysaccharides such as hyaluronic acid (HA); peptides; or a hydrophilic polymer composed of monomers of polyether such as polyethylene glycols (PEG), polyglycerols, polyoxazolines, polyols and aliphatic polyestesters. Preferably, the polymer provides stability and stealth properties to the lipid system and allows surface-decoration with ligands.

Preferably, the lipid-polymer or lipid-polymer derivatives are selected from the list consisting of any PEG-lipid compound or derivative, Polysarcosine-lipid or derivatives, polyoxazolines or derivatives, Lactosil-Derivatives. Preferably, the polymer comprised in the lipid-polymer is PEG, preferably DMG-PEG-2000. More preferably, the lipid-polymer or lipid-polymer derivative is selected from the list consisting of:

- DMG-PEG-2000 or DMG-PEG (1,2-dimyristoyl-rac-glycero-3-methoxypolyethylene glycol-2000),
- DSPE-PEG-2000 or DSPE-PEG (1,2-distearoyl-sn-glycero-3-phosphoethanolamine-N-[(polyethylene glycol)-2000]),
- DSG-PEG-2000 (1,2-Distearoyl-rac-glycero-3-methylpolyoxyethylene)
- DMPE-PEG or 14:0 PE PEG2000 (1,2-dimyristoyl-sn-glycero-3-phosphoethanolamine-N-[methoxy(polyethylene glycol)-2000]),
- DMPE-Psar(50) (1,2-dimyristoyl-sn-glycerol-3-phosphoethanolamine -Poly(sarcosine)50)
- VE-Psar (20) (Tocopoherol succinate isopropyl poly-sacrosine 20),
- PEG 2000 COOH or DSPE-PEG-2000 COOH or COOH (1,2-distearoyl-sn-glycero-3-phosphoethanolamine-N-[carboxy(polyethylene glycol)-2000] (sodium salt),
- PEG 2000 Maleimide or DSPE-PEG-2000 Maleimide or Mal (1,2-distearoyl-sn-glycero-3-phosphoethanolamine-N-[maleimide(polyethylene glycol)-2000] (ammonium salt)), and
- PEG 2000 DBCO or DSPE-PEG-2000 DBCO or DBCO (1,2-distearoyl-sn-glycero-3-phosphoethanolamine-N-[dibenzocyclooctyl(polyethylene glycol)-2000] (ammonium salt)).
- DMPE-Pox, DMPE-poly(2-oxazoline) or DMPE-poly(2-oxazoline) Maleinmide or DMPE-poly(2-oxazoline) DBCO.

In an embodiment, the lipid-polymer has spacers or linkers between the lipid molecule and the polymer derivative.

Preferably, the lipid-polymer is a pSAR lipid. As used herein "pSar (PolySarcosine) lipid" shall be understood as an amphiphilic molecule composed of a lipid head of any kind attached to a polymer composed of monomers of Sarcosine (also known as N-methylglycine), molecules. As used herein "DMPE-Psar" shall be understood as an amphiphilic molecule composed of a phospholipid, specifically DMPE, attached to a polymer composed of 'n' number of molecules of Sarcosine (also known as N-methylglycine).

Preferably, the lipid polymer or lipid-polymer derivative is selected from the list consisting of DSPE-PEG2000, DMG-PEG2000, DMPE-PSar and VE-PSar.

Even more preferably, the lipid polymer or lipid-polymer derivative is selected from the list consisting of DMPE-PSar (50) and VE-PSar (20).

In an embodiment, the lipid of the lipid-polymer or lipid-polymer derivative is a functionalized lipid-polymer or lipid-polymer derivative. As used herein "functionalized lipid-polymer or lipid-polymer derivative" shall be understood as a class of lipid-polymer derivative that has a specific functional group that is chemically active and capable of undergoing chemical reactions with other substances.

In the present invention, "functionalization" is understood as the union by means of chemical interactions (e.g. covalent, electrostatic, hydrophobic, etc,) of molecules that provide functionality to the lipid systems of the invention, whether they are drugs for therapeutic use, contrast elements for diagnostic use, ligands to mediate interactions with molecules or target cells, as well as charged compounds, surfactants or solvents to alter their physicochemical properties (size, dispersion and surface properties).

Preferably, the lipid-polymer or lipid-polymer derivative that is functionalized is selected from the list consisting of:

- DSPE-PEG 2000 COOH: 1,2-distearoyl-sn-glycero-3-phosphoethanolamine-N-[car-boxy(polyethylene glycol)-2000] (sodium salt),
- DSPE-PEG 2000 Maleimide: 1,2-distearoyl-sn-glycero-3-phosphoethanolamine-N-[maleimide(polyethylene glycol)-2000] (ammonium salt), and
- DSPE-PEG 2000 DBCO: 1,2-distearoyl-sn-glycero-3-phosphoethanolamine-N-[dibenzo-cyclooctyl(polyethylene glycol)-2000] (ammonium salt).

Preferably, the lipid-polymer or lipid-polymer derivative is functionalized or conjugated to one more ligands. In the present invention, "ligand" is understood as a molecule disposed towards the surface of the nanosystems, to favour the interaction thereof with target molecules, as receptors expressed at the level of the cell membrane of tumour cells. Depending on their nature, they can be nucleic acids, aptamers, peptides, proteins, hydrophilic or hydrophobic low molecular weight molecules, as well as derivatives thereof to generate amphiphilic molecules. In the present invention, "conjugates with ligands" means chemical conjugates that incorporate a ligand and a molecule with therapeutic activity (drug, radiopharmaceutical, etc.), or for diagnostic purposes (radioisotope, chelator, gadolinium, etc.).

In an embodiment, the lipid-polymer or lipid-polymer derivative is functionalized with at least one of the following elements:

- Therapeutic molecules;
- Contrast elements; or
- Ligands that mediate specific interactions with receptors and biomarkers.

Preferably, the lipid-polymer or lipid-polymer derivative is functionalized with at least one of the following elements:

- Drugs, preferably antitumor drugs such as carmofur, etoposide docetaxel, paclitaxel, gemcitabine, or derivatives thereof;
- Nucleic acids;
- Peptides;
- Proteins;
- targeting moieties (including antibody, anticalin, repebody, monobody, scFv, Fab, scFab, affibody, fynomer, DARPin, nanobody, or aptamers)
- Small organic molecules;
- Lipids with antitumor activity such as edelfosine;
- Compounds of natural origin with antitumor properties such as curcumin or resveratrol;
- Fluorophores such as green indiancyanine (also called ICG), 1,1-dioctadecyl-3,3,3,3-tetramethylindotricarbocyanine iodide (also called DiR), 1,1-dioctadecyl-3,3,3,3-tetramethylindodicarbocyanine perchlorate (also called DiD), nile Red or Alexa Fluor, 1,1'-Dioctadecyl-3,3,3',3'-Tetramethylindocarbocyanine Perchlorate (also called DIL), 3,3' -dioctadecyloxacarbocyanine, perchlorate (also called DiO), N-palmitoyl sphingomyelin-N (also called Cy5), N-[11-(dipyrrometheneboron difluoride)undecanoyl]-D-erythro-sphingosylphosphorylcholine (also called Top Fluor), N-palmitoyl sphingomyelin-N-(Cyanine 5);
- Inorganic nanoparticles such as superparamagnetic iron oxide particles (SPIONs);
- Chelating agents such as 1,4,7,10-Tetraazacyclododecane-1,4,7,10-tetraacetic acid (DOTA) or 1,2-distearoyl-sn-glycero-3-phosphoethanolamine-N-diethylenetriamine-pentaacetic acid (DTPA-PE) or 1,4,7-Triazacyclononane-1,4,7-triacetic acid (NOTE) or NOTE modified with a lipid for the complexation of gadolinium, or radioisotopes such as Gallium or Indium;
- other radioisotopes such as fluorine (18F), gallium (68Ga), iodine (125I), Indium (111In), and derivatives;
- Perfluorocarbons such as perfluorohexane and octafluoropropane;

as well as any combination of them.

Preferably, the lipid-polymer or lipid-polymer derivative is doubly functionalized with ligands suitable for cellular vehiculation and at least one therapeutic molecule or contrast element, as defined above.

e) <u>The at least one helper lipid.</u>

As explained above, the lipid system, preferably lipid nanosystem or lipid nanoparticle or population of lipid nanoparticles, may optionally comprise one or more helper lipids.

As used herein "helper lipid" shall be understood as a lipid and/or phospholipid or derivative thereof which plays an active role on formation, stability, efficacy, internalization and biodistribution of the claimed lipid systems.

Preferably, said helper lipid is selected from the list consisting of:

- 1.2-dioleoyl-sn-glycero-3-phosphoethanolamine (also called DOPE),
- 1,2-dioleoyl-3-trimethylammonium-propane (also called DOTAP),
- 1,2-dioleoyl-sn-glycero-3-phosphocholine (also called DOPC),
- 1-stearoyl-2-oleoyl-sn-glycero-3-phosphocholine (also called SOPC),
- 18/0/ 1,2-distearoyl-sn-glycero-3-phospho-L-serine (sodium salt) (also called phosphatidylserine or PS , and
- 1,2-distearoyl-sn-glycero-3-phosphate (also called 18PA).

More preferably, the helper lipid is SOPC or DOPE.

f) <u>The at least one surface-decoration moiety</u>

The lipid system, preferably lipid nanosystem or lipid nanoparticle or population of lipid nanoparticles, may optionally comprise one or more surface-decoration moieties.

As used herein "surface-decoration moiety" shall be understood as a molecule or functional group that is attached to the surface of a material to alter its properties. Surface decoration can be used to improve a material's adhesion, biocompatibility, and modulate biodistribution and cellular uptake, or other properties of the claimed lipid systems. Common types of surface-decoration moieties include organic molecules, peptides, proteins, and polymers, which can be incorporated by adsorption, establishment of electrostatic and covalent interactions. Moieties can also be modified moieties containing reactive groups such as azide and NHS groups, lipid-PEG derivatives in which the moiety of interest is linked, and other types of modifications aimed to favour disposition onto the surface of the claimed lipid systems. In some cases, the surface-decoration moiety is the helper lipid or the lipid-polymer, but in other cases the surface-decoration moiety is a completely different molecule that is also added to the lipid system of the invention.

Preferably, said moiety is selected from the list consisting of organic molecules, proteins, antibodies, affibodies, nucleic acids, aptamers, polymers and/or copolymers.

In an embodiment, the surface-decoration moiety comprises or consists of one or more organic molecules such as silanes, alkyl thiols, aromatic molecules, or derivatives.

In an embodiment, the surface-decoration moiety comprises or consists of one or more peptide or peptide derivatives (lipid-PEG-peptide or peptides with a reactive unit such as NHS or azide, among others), such as cell penetrating peptides, peptides with therapeutic properties and peptides able to recognize specific biomarkers, to interact with biological barriers or to modulate stability, cell internalization and biodistribution.

In an embodiment, the surface-decoration moiety comprises or consists of one or more antibodies or antibody derivatives (lipid-PEG-antibodies or antibodies with a reactive unit such as NHS or azide, linkers, or bifunctional linkers, among others), such as antibodies with therapeutic properties and antibodies able to recognize specific biomarkers, to interact with biological barriers or to modulate stability, cell internalization and biodistribution.

In an embodiment, the surface-decoration moiety comprises or consists of one or more affibodies or nanobodies or affibody or nanobody derivatives (lipid-PEG derivatives or moieties incorporating a reactive unit such as NHS or azide, linkers, or bifunctional linkers, among others), such as moieties able to recognize specific biomarkers, to interact with biological barriers or to modulate stability, cell internalization and biodistribution.

In an embodiment, the surface-decoration moiety comprises or consists of one or more proteins or protein derivatives (with a linker, a bifunctional linker, or a reactive unit for the establishment of covalent reactions such as NHS or azide, among others), such as proteins with therapeutic properties, proteins able to recognize specific biomarkers, to interact with biological barriers or to modulate stability, cell internalization and biodistribution.

In an embodiment, the surface-decoration moiety comprises or consists of one or more polymers, copolymers, or polymer and co-polymer derivatives (with a linker or a reactive unit for the establishment of covalent reactions such as NHS or azide, among others), such as polysaccharides, polylactic, polyglycolic acids, and combinations, polyaminoacids, stimuli-responsive polymers, stealth polymers, other, with therapeutic properties, able to recognize specific biomarkers, to interact with biological barriers or to modulate stability, cell internalization and biodistribution.

Each embodiment disclosed herein in items a) to d), preferably also e) and f), should be contemplated as being applicable to each of the other disclosed embodiments. Thus, all combinations of the various elements described herein are within the scope of the invention.

In an embodiment, the lipid system, preferably a lipid nanosystem or a lipid nanoparticles or population thereof, is characterized by comprising (components are separated by "/"):

- C12/VE/SM/DMG-PEG,
- C12/VE/SM/ DSPE-PEG,
- C12 /VE /SM/ DMPE-PSar (50),

- C12/VE/SM/VE-Psar (20),
- C12/VE/SM/DMPE-Pox,
- 306/VE/SM/DMG-PEG,
- 306/VE/SM/ DSPE-PEG,
- 306 /VE/ SM/ DMPE-PSar (50),
- 306/VE/SM/VE-Psar (20),
- 306/VE/SM/DMPE-Pox,
- MC3/VE/SM/DMG-PEG,
- MC3/VE/SM/ DSPE-PEG,
- MC3/VE /SM/ DMPE-PSar (50),
- MC3/VE/SM/VE-Psar (20),
- MC3/VE/SM/DMPE-Pox,
- C12/VE/SM/DOPE/DMG-PEG,
- C12/VE/SM/DOPE/DSPE-PEG,
- C12/VE/SM/DOPE/DMPE-Psar (50),
- C12/VE/SM/ DOPE/VE-Psar (20),
- C12/VE/SM/ DOPE/DMPE-Pox,
- 306/VE/SM/DOPE/DMG-PEG,
- 306/VE/SM/DOPE/ DSPE-PEG,
- 306/VE/SM/DOPE/ DMPE-Psar (50),
- 306/VE/SM/DOPE/ VE-Psar (20),
- 306/VE/SM/ DOPE/DMPE-Pox,
- MC3/VE/SM/DOPE/DMG-PEG,
- MC3/VE/SM/DOPE/ DSPE-PEG,
- MC3/VE/SM/DOPE/ DMPE-Psar (50),
- MC3/VE/SM/DOPE/ VE-Psar (20),
- MC3/VE/SM/ DOPE/DMPE-Pox,
- C12/VE/SM/ DOPC /DMG-PEG,
- C12/VE/SM/ DOPC /DSPE-PEG,
- C12/VE/SM/ DOPC /DMPE-Psar (50),
- C12/VE/SM/ DOPC /VE-Psar (20),
- C12/VE/SM/ DOPC /DMPE-Pox,
- 306/VE/SM/ DOPC /DMG-PEG,
- 306/VE/SM/ DOPC / DSPE-PEG,
- 306/VE/SM/ DOPC / DMPE-Psar (50),
- 306/VE/SM/ DOPC / VE-Psar (20),
- 306/VE/SM/ DOPC /DMPE-Pox,
- MC3/VE/SM/ DOPC /DMG-PEG,
- MC3/VE/SM/ DOPC / DSPE-PEG,
- MC3/VE/SM/ DOPC / DMPE-Psar (50),
- MC3/VE/SM/ DOPC / VE-Psar (20),
- MC3/VE/SM/ DOPC /DMPE-Pox,
- C12/VE/SM/ SOPC /DMG-PEG,
- C12/VE/SM/ SOPC /DSPE-PEG,
- C12/VE/SM/ SOPC /DMPE-Psar (50),
- C12/VE/SM/ SOPC /VE-Psar (20),
- C12/VE/SM/ SOPC /DMPE-Pox,
- 306/VE/SM/ SOPC /DMG-PEG,
- 306/VE/SM/ SOPC / DSPE-PEG,
- 306/VE/SM/ SOPC / DMPE-Psar (50),
- 306/VE/SM/ SOPC / VE-Psar (20),
- 306/VE/SM/ SOPC /DMPE-Pox,
- MC3/VE/SM/ SOPC /DMG-PEG,
- MC3/VE/SM/ SOPC / DSPE-PEG,
- MC3/VE/SM/ SOPC / DMPE-Psar (50),
- MC3/VE/SM/ SOPC / VE-Psar (20),
- MC3/VE/SM/ SOPC /DMPE-Pox,
- C12/OleicAcid/SM/DMG-PEG,

- C12/OleicAcid/SM/DMPE-Psar(50)
- C12/OleicAcid/SM/VEsar(20)
- C12/Mygliol/SM/DMG-PEG,
- C12/Mygliol/SM/DMPE-Psar(50)
- C12/Mygliol/SM/VE-Psar(20)
- 306/OleicAcid/SM/DMG-PEG,
- 306/OleicAcid/SM/DMPE-Psar(50)
- 306/OleicAcid/SM/VEsar(20)
- 306/Mygliol/SM/DMG-PEG,
- 306/Mygliol/SM/DMPE-Psar(50)
- 306/Mygliol/SM/VE-Psar(20)
- C12/OleicAcid/SM/DOPE/DMG-PEG,
- C12/OleicAcid/SM/DOPE/DMPE-Psar(50)
- C12/OleicAcid/SM/DOPE/VE-Psar(20)
- C12/Mygliol/SM/DOPE/DMG-PEG,
- C12/Mygliol/SM/DOPE/DMPE-Psar(50)
- C12/Mygliol/SM/DOPE/VE-Psar(20),
- 306/OleicAcid/SM/DOPE/DMG-PEG,
- 306/OleicAcid/SM/DOPE/DMPE-Psar(50)
- 306/OleicAcid/SM/DOPE/VE-Psar(20)
- 306/Mygliol/SM/DOPE/DMG-PEG,
- 306/Mygliol/SM/DOPE/DMPE-Psar(50)
- 306/Mygliol/SM/DOPE/VE-Psar(20),
- C12/VE/SM/ DOTAP /DSPE-PEG,
- C12/VE/SM/ DOTAP /DMPE-Psar (50),
- C12/VE/SM/ DOTAP /VE-Psar (20),
- C12/VE/SM/ DOTAP / DMPE-Pox,
- 306/VE/SM/ DOTAP /DMG-PEG,
- 306/VE/SM/ DOTAP / DSPE-PEG,
- 306/VE/SM/ DOTAP / DMPE-Psar (50),
- 306/VE/SM/ DOTAP / VE-Psar (20),
- 306/VE/SM/ DOTAP / DMPE-Pox,
- C12/VE/SM/ DOTAP /DMG-PEG,
- C12/VE/SM/ DOTAP /DMG-PEG,
- MC3/VE/SM/DMG-PEG,
- MC3/VE/SM/ DOTAP /DSPE-PEG,
- MC3/VE/SM/ DOTAP /DMPE-Psar (50),
- MC3/VE/SM/ DOTAP /VE-Psar (20),
- MC3/VE/SM/ DOTAP / DMPE-Pox,
- DLinDMA/VE/SM/DMG-PEG,
- SM-102/VE/SM/DMG-PEG,
- ALC-0315/VE/SM/DMG-PEG,
- C12/VE/SM/DOPC/DMG-PEG/DBCO,
- 306/VE/SM/DOPC/DMG-PEG/DBCO,
- C12/VE/SM/SOPC/DMG-PEG/DBCO,
- 306/VE/SM/SOPC/DMG-PEG/DBCO,
- C12/VE/SM/SOPC/DMG-PEG/MAL,
- 306/VE/SM/SOPC/DMG-PEG/MAL,
- SM-102/VE/SM/DOPC/DMG-PEG/DBCO,
- SM-102/VE/SM/SOPC/DMG-PEG/DBCO,
- SM-102/VE/SM/DOPE/DMG-PEG/DBCO,
- SM-102/VE/SM/DOPC/DMG-PEG/MAL,
- SM-102/VE/SM/SOPC/DMG-PEG/MAL,
- SM-102/VE/SM/DOPE/DMG-PEG/MAL,
- ALC-0315/VE/SM/DOPC/DMG-PEG/DBCO,
- ALC-0315/VE/SM/SOPC/DMG-PEG/DBCO,
- ALC-0315/VE/SM/DOPE/DMG-PEG/DBCO,
- ALC-0315/VE/SM/SOPC/DMG-PEG/MAL,

- ALC-0315/VE/SM/SOPC/DMG-PEG/MAL,
- ALC-0315 /VE /SM /DOPE /DMG-PEG /MAL.

Preferably, the lipid system, preferably a lipid nanosystem or a lipid nanoparticles or population thereof, is characterized by comprising (components are separated by "/"):

- C12/VE/SM/DOPE/DMG-PEG, or
- 306/VE/SM/DOPE/DMG-PEG
- 306NE/SM/DOPE/DM PE-Psar(50)
- C12/VE/SM/DMG-PEG
- C12/VE/SM/DMPE-Psar(50)

It is noted that in the list above and through the entire document, the following abbreviations are used:

- Vitamin E is also referred to as VE;
- Sphingomyelin is also referred to as SM;
- C12-200 is also referred to as C12;
- 306-O12-B is also referred to as 306;
- PEG-Lipid stands for any PEG-lipid compound or derivative disclosed;
- DMG-PEG-2000 is also referred to as DMG-PEG or DMG-PEG-2k;
- DSPE-PEG-2000 is also referred to as DSPE-PEG-2k
- DMPE-Psar is also referred to as PE-Psar;
- VE-Psar is also referred to as VE-Psar
- DMPE-Psar(50) is also referred to as PE-Psar having a polymer of 50 sarcosine molecules;
- VE-Psar(20) is also referred to as VE-Psar having a polymer of 20 sarcosine molecules;
- DSPE-PEG-2000 Maleimide is also referred to as Mal or Maleimide or PEG-Maleimide;
- DSPE-PEG-2000 DBCO is also referred to as DBCO or PEG-DBCO.

Preferably, the lipid system, preferably lipid nanosystem or lipid nanoparticle or population of lipid nanoparticles, is characterized by comprising vitamin E and sphingomyelin, a lipid-polymer, and at least one ionizable lipid. Preferably, the lipid system, preferably lipid nanosystem or lipid nanoparticle or population of lipid nanoparticles, is characterized by comprising mygliol, sphingomyelin, a lipid-polymer and at least one ionizable lipid. Preferably, the lipid system, preferably lipid nanosystem or lipid nanoparticle or population of lipid nanoparticles, is characterized by comprising oleic acid, sphingomyelin, a lipid-polymer and at least one ionizable lipid.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DMG-PEG-2000,
- vitamin E, sphingomyelin, C12-200 and DMPE-PEG-2000,
- vitamin E, sphingomyelin, C12-200, and DMPE-PSar, preferably DMPE-Psar (50),
- vitamin E, sphingomyelin, C12-200, and VE-PSar, preferably VE-Psar(20),
- vitamin E, sphingomyelin, C12-200, and DSPE-PEG-2000,
- vitamin E, sphingomyelin, 306-O12B, and DMG-PEG-2000,
- vitamin E, sphingomyelin, 306-O12B, and DSPE-PEG-2000,
- vitamin E, sphingomyelin, 306-O12B, and DMPE-PEG-2000,
- vitamin E, sphingomyelin, 306-O12B, and DMPE-PSar preferably DMPE-Psar(50),
- vitamin E, sphingomyelin, 306-O12B, and VE-PSar preferably VE-Psar(20),
- Oleic Acid, sphingomyelin, C12-200, DMG-PEG,
- Oleic Acid, sphingomyelin, C12-200, DMPE-Psar, preferably DMPE-Psar(50)
- Oleic Acid, sphingomyelin, C12-200, VE-Psar, preferably VE-Psar (20)
- Mygliol, sphingomyelin, C12-200, DMG-PEG;
- Mygliol, sphingomyelin, C12-200, DMPE-Psar, preferably DMPE-Psar (50);
- Mygliol, sphingomyelin, C12-200, VE-Psar, preferably VE-Psar(20);

and wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DMG-PEG-2000,
- vitamin E, sphingomyelin, C12-200, and DMPE-PSar preferably DMPE-Psar (50),
- vitamin E, sphingomyelin, C12-200, and VE-PSar, preferably VE-Psar (20),
- vitamin E, sphingomyelin, C12-200, and DSPE-PEG-2000,
- vitamin E, sphingomyelin, C12-200, and DMPE-PEG-2000,
- vitamin E, sphingomyelin, 306-O12B, and DMG-PEG-2000,
- vitamin E, sphingomyelin, 306-O12B, and DSPE-PEG-2000,
- vitamin E, sphingomyelin, 306-O12B, and DMPE-PSar preferably DMPE-Psar (50),
- vitamin E, sphingomyelin, 306-O12B, and DMPE-PEG-2000,
- vitamin E, sphingomyelin, 306-O12B, and VE-PSar, preferably VE-Psar (20),
- Oleic Acid, sphingomyelin, C12-200, DMG-PEG,
- Oleic Acid, sphingomyelin, C12-200, DMPE-Psar, preferably DMPE-Psar(50),
- Oleic Acid, sphingomyelin, C12-200, VE-Psar, preferably VE-Psar(20), or
- Mygliol, sphingomyelin, C12-200, DMG-PEG;
- Mygliol, sphingomyelin, C12-200, DMPE-Psar (50);
- Mygliol, sphingomyelin, C12-200, VE-Psar(20);

wherein the system further comprises a helper lipid such as DOPE, DOPC, or SOPC; and wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DMG-PEG-2000,
- vitamin E, sphingomyelin, C12-200, and DMPE-PSar, preferably DMPE-Psar (50),
- vitamin E, sphingomyelin, C12-200, and VE-PSar, preferably VE-Psar (20),
- vitamin E, sphingomyelin, C12-200, and DSPE-PEG-2000,
- vitamin E, sphingomyelin, C12-200, and DMPE-PEG-2000,
- vitamin E, sphingomyelin, 306-O12B, and DMG-PEG-2000,
- vitamin E, sphingomyelin, 306-O12B, and DSPE-PEG-2000,
- vitamin E, sphingomyelin, 306-O12B, and DMPE-PSar, preferably DMPE-Psar (50),
- vitamin E, sphingomyelin, 306-O12B, and DMPE-PEG-2000,
- vitamin E, sphingomyelin, 306-O12B, and VE-PSar, preferably VE-Psar(20),
- Oleic Acid, sphingomyelin, C12-200, DMG-PEG,
- Oleic Acid, sphingomyelin, C12-200, DMPE-Psar, preferably DMPE-Psar(50),
- Oleic Acid, sphingomyelin, C12-200, VE-Psar, preferably VE-Psar(20)
- Mygliol, sphingomyelin, C12-200, DMG-PEG;
- Mygliol, sphingomyelin, C12-200, DMPE-Psar, preferably DMPE-Psar(50) or,

- Mygliol, sphingomyelin, C12-200, VE-Psar, preferably VE-Psar(20)

wherein the system further comprises SOPC as a helper lipid; and wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DMG-PEG-2000,
- vitamin E, sphingomyelin, C12-200, and DMPE-PSar, preferably DMPE-Psar(50),
- vitamin E, sphingomyelin, C12-200, and VE-PSar, preferably VE-Psar(20),
- vitamin E, sphingomyelin, C12-200, and DSPE-PEG-2000,
- vitamin E, sphingomyelin, C12-200, and DMPE-PEG-2000,
- vitamin E, sphingomyelin, 306-O12B, and DMG-PEG-2000,
- vitamin E, sphingomyelin, 306-O12B, and DSPE-PEG-2000,
- vitamin E, sphingomyelin, 306-O12B, and DMPE-PSar, preferably DMPE-Psar(50),
- vitamin E, sphingomyelin, 306-O12B, and DMPE-PEG-2000,
- vitamin E, sphingomyelin, 306-O12B, and VE-PSar, preferably VE-Psar(20),
- Oleic Acid, sphingomyelin, C12-200, DMG-PEG,
- Oleic Acid, sphingomyelin, C12-200, DMPE-Psar, preferably DMPE-Psar(50),
- Oleic Acid, sphingomyelin, C12-200, VE-Psar, preferably VE-Psar(20),
- Mygliol, sphingomyelin, C12-200, DMG-PEG,
- Mygliol, sphingomyelin, C12-200, DMPE-Psar, preferably DMPE-Psar(50) or
- Mygliol, sphingomyelin, C12-200, VE-Psar, preferably VE-Psar(20),

wherein the system further comprises DOPE as a helper lipid; and wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, C12-200, DOPE, and DMG-PEG-2000,
- vitamin E, sohingomyelin, C12-200, DOPE and DSPE-PEG-2000,
- vitamin E, sphingomyelin, C12-200, DOPE, and DMPE-PSar(50),
- vitamin E, sphingomyelin, C12-200, DOPE, and VE-PSar(20),
- vitamin E, sphingomyelin, C12-200, DOPE, and DSPE-PEG-2000
- vitamin E, sphingomyelin, 306-O12B, DOPE, and DMG-PEG-2000,
- vitamin E, sphingomyelin, 306-O12B, DOPE, and DSPE-PEG-2000,
- vitamin E, sphingomyelin, 306-O12B, DOPE, and DMPE-PSar(50), or
- vitamin E, sphingomyelin, 306-O12B, DOPE, and VE-PSar(20), and wherein, preferably:
    - the lipid system does not comprise cholesterol, or
    - the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, C12-200, DOPC, and DMG-PEG-2000,
- vitamin E, sohingomyelin, C12-200, DOPC and DSPE-PEG-2000,

- vitamin E, sphingomyelin, C12-200, DOPC, and DMPE-PSar(50),
- vitamin E, sphingomyelin, C12-200, DOPC, and VE-PSar(20),
- vitamin E, sphingomyelin, C12-200, DOPC, and DSPE-PEG-2000
- vitamin E, sphingomyelin, 306-O12B, DOPC, and DMG-PEG-2000,
- vitamin E, sphingomyelin, 306-O12B, DOPC, and DSPE-PEG-2000,
- vitamin E, sphingomyelin, 306-O12B, DOPC, and DMPE-PSar(50), or
- vitamin E, sphingomyelin, 306-O12B, DOPC, and VE-PSar(20), and

wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, C12-200, SOPC, and DMG-PEG-2000,
- vitamin E, sohingomyelin, C12-200, SOPC and DSPE-PEG-2000,
- vitamin E, sphingomyelin, C12-200, SOPC, and DMPE-PSar(50),
- vitamin E, sphingomyelin, C12-200, SOPC, and VE-PSar(20),
- vitamin E, sphingomyelin, C12-200, SOPC, and DSPE-PEG-2000
- vitamin E, sphingomyelin, 306-O12B, SOPC, and DMG-PEG-2000,
- vitamin E, sphingomyelin, 306-O12B, SOPC, and DSPE-PEG-2000,
- vitamin E, sphingomyelin, 306-O12B, SOPC, and DMPE-PSar(50), or
- vitamin E, sphingomyelin, 306-O12B, SOPC, and VE-PSar(20), and

wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising vitamin E, sphingomyelin, C12-200, SOPC and PEG2000-Maleimide, wherein preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising vitamin E, sphingomyelin, C12-200, DOPE and PEG2000-Maleimide, wherein preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DMG-PEG-2000,
- vitamin E, sphingomyelin, C12-200, and DMPE-PSar, preferably DMPE-Psar(50),
- vitamin E, sphingomyelin, C12-200, and VE-PSar, preferably VE-PSAR(20),
- vitamin E, sphingomyelin, C12-200, and DSPE-PEG-2000,
- vitamin E, sphingomyelin, C12-200, and DMPE-PEG-2000,
- vitamin E, sphingomyelin, 306-O12B, and DMG-PEG-2000,
- vitamin E, sphingomyelin, 306-O12B, and DSPE-PEG-2000,

- vitamin E, sphingomyelin, 306-O12B, and DMPE-PSar(50),
- vitamin E, sphingomyelin, 306-O12B, and DMPE-PEG-2000,
- vitamin E, sphingomyelin, 306-O12B, and VE-PSar(20),
- Oleic Acid, sphingomyelin, C12-200, DMG-PEG, or
- Mygliol, sphingomyelin, C12-200, DMG-PEG.

wherein the system further comprises a functionalized lipid, preferably PEG2000-DBCO or PEG2000-Maleimide; and wherein, preferably:

- the lipid system does not comprise cholesterol, or
- the lipid system does not comprise cholesterol nor DSPC.

Preferably, the lipid system, preferably lipid nanosystem or lipid nanoparticle or population of lipid nanoparticles, is characterized by comprising:

- vitamin E, sphingomyelin, at least one ionizable lipid and at least one lipid-polymer,
- mygliol, sphingomyelin, at least one ionizable lipid and at least one lipid-polymer,
- oleic acid, sphingomyelin, at least one ionizable lipid and at least one lipid-polymer,
- vitamin E, sphingomyelin, C12-200 and at least one lipid-polymer,
- vitamin E, sphingomyelin, 306-O12B and at least one lipid-polymer,
- vitamin E, sphingomyelin, C12-200, and DSPE-PEG2000-DBCO,
- vitamin E, sphingomyelin, C12-200, and DSPE-PEG2000-Maleimide,
- vitamin E, sphingomyelin, 306-O12B, and DSPE-PEG2000-DBCO, or
- vitamin E, sphingomyelin, 306-O12B, and DSPE-PEG2000-Maleimide;

and wherein, preferably:

- the lipid system does not comprise cholesterol, or
- the lipid system does not comprise cholesterol nor DSPC.

In an embodiment, if the lipid system comprises vitamin E and sphingomyelin, at least an ionizable lipid and at least one lipid-polymer, then the lipid system does not comprise cholesterol nor DSPC.

In an embodiment, if the lipid system comprises DSPE-PEG2000-Maleimide, preferably vitamin E, sphingomyelin and DSPE-PEG2000-Maleimide, then the lipid system does not comprise cholesterol.

In an embodiment, if the lipid system comprises DSPE-PEG2000-DBCO, preferably vitamin E, sphingomyelin and DSPE-PEG2000-DBCO, then the lipid system does not comprise cholesterol.

Preferably, the lipid system, preferably lipid nanosystem or lipid nanoparticle or population of lipid nanoparticles, is characterized by comprising:

| SNPs name | COMPOUNDS COMBINATION |
|---|---|
| SM/DOPE | C12/VE/SM/DOPE/DMG-PEG |
| SM VE DOPC | C12/VE/SM/DOPC/DMG-PEG |
| SMx2 18PA | C12/VE/SM/18PA/DMG-PEG |
| SMx2 DOTAP | C12/VE/SM/DOTAP/DMG-PEG |
| SMx2 DMPE Psar | C12/VE/SM/DMPE-Psar |
| SMx2 VE Psar | C12/VE/SM/VE-Psar |
| 306 SM | 306/VE/SM/DMG-PEG |
| 306 SMx2 DOPE | 306/VE/SM/DOPE/DMG-PEG |
| **306** DOPE | 306/VE/SM/DOPE/DMPE-Psar |
| **306** DOPE VE/Psar | 306/VE/SM/DOPE/VE-Psar |
| **306** DOPE | 306/VE/SM/DOPE/DMPE-Psar |

(continued)

| | |
|---|---|
| MC3 SM | MC3/VE/SM/DMG-PEG |
| DLin-DMA SM | DLinDMA/VE/SM/DMG-PEG |
| C12.1-3 | C12/VE/SM/DOPC/DMG-PEG/DBCO |
| C12.4-6 | C12/VE/SM/SOPC/DMG-PEG/DBCO |
| C12.7-9 | C12/VE/SM/DOPE/DMG-PEG/DBCO |
| C12.11,13 | C12/VE/SM/SOPC/DMG-PEG/MAL |
| SM-102.1-3 | SM-102/VE/SM/DOPC/DMG/DBCO |
| SM-102.4-6 | SM-102/VE/SM/SOPC/DMG/DBCO |
| SM-102.7-8 | SM-102/VE/SM/DOPE/DMG/DBCO |
| SM-102.10-12 | SM-102/VE/SM/SOPC/DMG/MAL |
| ALC-0315.1-3 | ALC-0315/VE/SM/SOPC/DMG/DBCO |
| ALC-0315.5,7 | ALC-0315/VE/SM/SOPC/DMG/MAL |

| | |
|---|---|
| 306.2,4 | 306/VE/SM/SOPC/DMG-PEG/MAL |
| C12-OleicAcid 1 | C12/OleicAcid/SM/DMG-PEG |
| C12-OleicAcid 2 | C12/OleicAcid/SM/DMPE-Psar(50) |
| C12-OleicAcid 3 | C12/OleicAcid/SM/VE-Psar(20) |
| C12-OleicAcid 4 | C12/OleicAcid/SM/DMG-PEG |
| C12-OleicAcid 5 | C12/OleicAcid/SM/DMPE-Psar(50) |
| C12-OleicAcid 6 | C12/OleicAcid/SM/VE-Psar(20) |
| 306-OleicAcid 1 | 306/OleicAcid/SM/DMG-PEG |
| 306-OleicAcid 2 | 306/OleicAcid/SM/DMPE-Psar(50) |
| 306-OleicAcid 3 | 306/OleicAcid/SM/VE-Psar(20) |
| 306-OleicAcid 4 | 306/OleicAcid/SM/DMG-PEG |
| 306-OleicAcid 5 | 306/OleicAcid/SM/DMPE-Psar(50) |
| 306-OleicAcid 6 | 306/OleicAcid/SM/VE-Psar(20) |
| C12-Miglyol 1 | C12/ Miglyol /SM/DMG-PEG |
| C12-Miglyol 2 | C12/ Miglyol /SM/DMPE-Psar(50) |
| C12-Miglyol 3 | C12/ Miglyol /SM/VE-Psar(20) |
| C12- Miglyol 4 | C12/ Miglyol /SM/DMG-PEG |
| C12- Miglyol 5 | C12/ Miglyol /SM/DMPE-Psar(50) |
| C12- Miglyol 6 | C12/ Miglyol/SM/VE-Psar(20) |
| 306- Miglyol 1 | 306/ Miglyol /SM/DMG-PEG |
| 306- Miglyol 2 | 306/ Miglyol /SM/DMPE-Psar(50) |
| 306- Miglyol 3 | 306/ Miglyol/SM/VE-Psar(20) |
| 306- Miglyol 4 | 306/ Miglyol /SM/DMG-PEG |
| 306- Miglyol 5 | 306/ Miglyol /SM/DMPE-Psar(50) |
| 306- Miglyol 6 | 306/ Miglyol/SM/VE-Psar(20) |

The lipid system may be modulated by modifying the ratios of each of components a) to d), and optionally e).

Preferred ratios are:

- the oil of a), preferably Vitamin E, is at a molar ratio percentage between 2 and 90,
- the ionizable lipid of b), preferably C12-200 or 306-O12B, is at a molar ratio percentage between 10 and 90,
- the sphingolipid of c), preferably sphingomyelin, is at a molar ratio percentage between 2 and 90, or
- the lipid-polymer of d) is at a molar ratio percentage between 0.1 and 5

and wherein, preferably, the helper lipid of e) is at a molar concentration between 5 and 80.

Further, the relative amount of oil with respect to Sphingomyelin may also be varied in order to modulate the lipid system's features and to modulate it to the interest of the specific application, as shown in the Examples with Vitamin E and SM.

Preferably, the lipid system, preferably lipid nanosystem or lipid nanoparticle or population of lipid nanoparticles, comprises vitamin E and sphingomyelin, wherein the amount of sphingomyelin is superior to the amount of vitamin E. Preferably, the amount of sphingomyelin is at least double of the amount of vitamin E. Preferably, the amount of sphingomyelin is at least four times higher than the amount of vitamin E.

Preferably, the lipid system, preferably lipid nanosystem or lipid nanoparticle or population of lipid nanoparticles, comprises C12, SM, and Oleic acid. Preferably, the lipid system, preferably lipid nanosystem or lipid nanoparticle or population of lipid nanoparticles, comprises C12, SM, and mygliol.

Other elements of the system.

As shown in the Examples, the lipid system, preferably lipid nanosystem or lipid nanoparticle or population of lipid nanoparticles, may comprise other elements or molecules. This is because the lipid system can be functionalized so that it acts as a vehicle to deliver said other elements to a target of interest, such as to a target cell or tissue.

In an embodiment, the lipid system further comprises, for example loaded or encapsulated, one or more nucleic acid molecules or nucleotide complexes such as a ribonucleoprotein (RNP) complex. In an embodiment, the system comprises mRNAs. siRNAs, ASOs, pDNA, aptamers, IncRNA, and/or CRISPR/Cas genome editing systems. In an embodiment, the lipid system further comprises proteins, such as peptides, polypeptides and/or antibodies or targeting moieties.

In some embodiments, the lipid system comprises in its surface ligands, peptides, proteins, nucleic acids and/or antibodies.

In **a second aspect,** the composition of the first aspect, or any of its embodiments, is a pharmaceutical composition and optionally comprises excipients and/or carriers.

A **third aspect** of the invention relates to the pharmaceutical composition of the second aspect, for use in therapy. In an embodiment, the use in therapy is a use for delivery CRISPR/Cas genome editing components in a subject in need thereof. In an embodiment, the use in therapy is a use for delivery a target protein or nucleic acid molecule to a subject in need thereof. Preferably, the use is for mRNA delivery.

Other uses include the treatment of cancer, in particular of breast cancer, melanoma, uveal melanoma, pancreatic cancer, lung cancer, prostate cancer, stomach cancer, head and neck cancer, sarcoma, glioblastoma, neuroblastoma, cancer of the colon and rectum, cancer of the head and neck, kidney and bladder cancer, and hepatocarcinoma. More particularly, for its use in the treatment of cancer metastasis. The use also includes the use in diagnosis.

Other uses also included herein are in advanced cell therapies, regenerative medicine, rare diseases, metabolic diseases, inflammatory diseases, degenerative diseases, vaccines, or autoimmune disorders.

Preferably, the use implies the administration of the lipid system of the invention or the pharmaceutical composition in a subject in need thereof.

The composition of the invention can be administered by any appropriate route which results in an effective treatment in the subject in need thereof, including, but not limited to, orally or parenteral routes, including intravenous, intraarterial, intrathecal, intramuscular, transdermally, topical, subcutaneous, transdermal, airway, intraarticular, intrathecal, nasal, rectal. Preferably, the use implies intravenous administration.

The invention is described below by the following examples. which must be considered as merely illustrative and in no case limiting of the scope of the present invention.

The present invention also provides the following clauses:

1. A composition comprising a lipid system, preferably a lipid nanosystem or a lipid nanoparticle, wherein the lipid system is characterized by comprising:

   a) at least one oil,
   b) at least one ionizable lipid,
   c) at least one sphingolipid, and

d) at least one lipid-polymer,

preferably with the proviso is that the lipid system does not comprise cholesterol.

2. The composition according to clause 1, wherein the at least one oil is selected from the list consisting of α-tocopherol (vitamin E), mygliol, Olive (oleic acid), Sunflower oil, Peanut, Avocado, Argan, Almond, Calendula, Coconut, Wheat germ, Arnica, Borage, Sesame, Cotton, Castor bean, Safflower, Palm, Wheat germ, Tea tree, Jojoba, Linseed, Silicone, Glycerol, Triglyceride oils, Hyperi-cum, Rosehip, and Isopropyl, squalene, tributyrin, or any combination there-of.

3. The composition according to clause 1 or 2, wherein the at least one ionizable lipid is selected from the list consisting of:

- N,N-dimethyl-2,3-bis[(9Z)-9-octadecen-1-yloxy]-1-propanamine (DODMA),
- N,N-dimethyl-2,3-bis[(9Z,12Z)-9,12-octadecadien-1-yloxy]-1-propanamine (DLin-DMA) lipid),
- N,N-dimethyl-2,2-di-(9Z,12Z)-9,12-octadecadien-1-yl-1,3-dioxolane-4-ethanamine (DLin-KC2-DMA),
- 4-(dimethylamino)-butanoic acid, (10Z,13Z)-1-(9Z,12Z)-9,12-octadecadien-1-yl-10,13-nonadecadien-1-yl ester (DLin-MC3-DMA lipid),
- 8-[(2-hydroxyethyl)[6-oxo-6-(undecyloxy)hexyl]amino]-octanoic acid, 1-octylnonyl ester (SM-102),
- 2-hexyl-decanoic acid, 1,1'-[[(4-hydroxybutyl)imino]di-6,1-hexanediyl] ester (ALC-0315),
- 1,1'-[[2-[4-[2-[[2-[bis(2-hydroxydodecyl)amino]ethyl](2-hydroxydodecyl)amino]ethyl]-1-piperazinyl]ethyl]imino] bis-2-dodecanol (C12-200),
- 3,6-bis[4-[bis(2-hydroxydodecyl)amino]butyl]-2,5-piperazinedione (CKK-E12),
- tetrakis(8-methylnonyl) 3,3',3",3'''-(((methylazanediyl)bis(propane-3,1-diyl))bis(azanetriyl))tetrapropionate (306-Oi10),
- 2-(dioctylamino)ethyl nonyl hydrogen phosphate (9A1P9),
- tetrakis(2-(octyldisulfaneyl)ethyl) 3,3',3",3'''-(((methylazanediyl)bis(propane-3,1-diyl))bis(azanetriyl))tetrapropionate (306-O12B),
- 1,1',1",1'''-[(3,6-dioxo-2,5-piperazinediyl)bis(4,1-butanediylnitrilodi-2,1-ethanediyl)] ester (OF-Deg-Lin, 9Z.12Z-octadecadienoic acid ), and
- 9-[4-(dimethylamino)-1-oxobutoxy]-heptadecanedioic acid, 1,17-di-(2Z)-2-nonen-1 (L319).

4. The composition according to any one of clauses 1 to 3, wherein the at least one sphingolipid is selected from the list consisting of sphingomyelin, sphingosine, Phosphosphyngolipids glycosphingolipid and ceramide.

5. The composition according to any one of clauses 1 to 4, wherein the at least one lipid-polymer is selected from the list consisting of PEG-lipid, Polysarco-sine, or Lactosil-Derivatives.

6. The composition according to any one of clauses 1 to 5, wherein at least one oil is selected from the list consisting of α-tocopherol (vitamin E), oleic acid, linoleic acid and/or mygliol.

7. The composition according to any one of clauses 1 to 6, wherein the at least one ionizable lipid is selected from the list consisting of:

- tetrakis(2-(octyldisulfaneyl)ethyl) 3,3',3",3'''-(((methylazanediyl)bis(propane-3,1-diyl))bis(azanetriyl))tetrapropionate (306-O12B), or
- 1,1'-[[2-[4-[2-[[2-[bis(2-hydroxydodecyl)amino]ethyl](2-hydroxydodecyl)amino]ethyl]-1-piperazinyl]ethyl]imino] bis-2-dodecanol (C12-200).

8. The composition according to any one of clauses 1 to 7, wherein the at least one sphingolipid is sphingomyelin.

9. The composition according to any one of clauses 1 to 8, wherein the at least one lipid-polymer is selected from the list consisting of DSPE-PEG2000 (1,2-distearoyl-sn-glycero-3-phosphoethanolamine-N-[(polyethylene glycol)-2000]), DMG-PEG2000 (1.2-dimyristoyl-rac-glycero-3-methoxypolyethylene glycol-2000), DMPE-PSar (50) (1,2-dimyristoyl-sn-glycerol-3-phosphoethanolamine -Poly(sarcosine)50) and VE-PSar (20) (To-copoherol succinate isopropyl poly-sacrosine 20).

10. The composition according to any one of clauses 1 to 9, wherein the at least one oil is vitamin E and the at least one

ionizable lipid is C12-200.

11. The composition according to any one of clauses 1 to 10, wherein the at least one oil is vitamin E, the at least one ionizable lipid is C12-200, and the at least one lipid-polymer is DMG-PEG-2000.

12. The composition according to any one of clauses 1 to 10, wherein the at least one oil is vitamin E, the at least one ionizable lipid is C12-200, and the at least one lipid-polymer is DMPE-Psar(50).

13. The composition according to any one of clauses 1 to 10, wherein the at least one oil is vitamin E, the at least one ionizable lipid is C12-200, and the at least one lipid-polymer is VE-Psar(20).

14. The composition according to any one of clauses 1 to 9, to wherein the at least one oil is vitamin E and the at least one ionizable lipid is 306-O12B.

15. The composition according to any one of clauses 1 to 9, wherein the at least one oil is vitamin E and the at least one ionizable lipid is 306-O12B, and the at least one lipid-polymer is DMPE-Psar(50).

16. The composition according to any one of clauses 1 to 9, wherein the at least one oil is vitamin E and the at least one ionizable lipid is 306-O12B, and the at least one lipid-polymer is VE-Psar(20).

17. The composition according to any one of clauses 1 to 9, wherein the at least one lipid-polymer is selected from the list consisting of:

- PEG 2000 COOH: 2-distearoyl-sn-glycero-3-phosphoethanolamine-N-[carboxy(polyethylene glycol)-2000] (sodium salt),
- PEG 2000 Maleimide: 1,2-distearoyl-sn-glycero-3-phosphoethanolamine-N-[maleimide(polyethylene glycol)-2000] (ammo-nium salt), and
- PEG 2000 DBCO: 1,2-distearoyl-sn-glycero-3-phosphoethanolamine-N-[dibenzocyclooctyl(polyethylene glycol)-2000] (ammonium salt).

18. The composition according to any one of clauses 10 to 17, wherein the at least one sphingolipid is sphingomyelin.

19. The composition according to any one of clauses 1 to 18, wherein the composition further comprises a e) at least one helper lipid and/or f) at least one sur-face-decoration moiety.

20. The composition according to clause 19, wherein the at least one helper lipid is selected from the list consisting of:

- 1.2-dioleoyl-sn-glycero-3-phosphoethanolamine (DOPE),
- 1,2-distearoyl-sn-glycero-3-phosphocholine (DSPC),
- 1,2-dioleoyl-3-trimethylammonium-propane (DOTAP),
- 1,2-dioleoyl-sn-glycero-3-phosphocholine (DOPC),
- 1-stearoyl-2-oleoyl-sn-glycero-3-phosphocholine (SOPC),
- 18/0/ 1,2-distearoyl-sn-glycero-3-phospho-L-serine (sodium salt) (phos-phatidylserine or PS), and
- 1,2-distearoyl-sn-glycero-3-phosphate (also called 18PA).

21. The composition according to any one of clauses 19 or 20, wherein the at least one oil is vitamin E, the at least one ionizable lipid is C12-200, the at least one sphingolipid is sphingomyelin, the at least one lipid-polymer is DMG-PEG-2000, and the at least a helped lipid is DOPE.

22. The composition according to any one of clauses 19 or 20, wherein the at least one oil is vitamin E, the at least one ionizable lipid is 306-O12B, the at least one sphingolipid is sphingomyelin, the at least one lipid-polymer is DMG-PEG-2000, and the at least a helped lipid is DOPE.

23. The composition according to any one of clauses 19 or 20, wherein the at least one oil is vitamin E, the at least one ionizable lipid is C12-200, the at least one sphingolipid is sphingomyelin, the at least one lipid-polymer is DMG-PEG-2000.

24. The composition according to any one of clauses 19 or 20, wherein the at least one oil is vitamin E, the at least one

ionizable lipid is 306-O12B, the at least one sphingolipid is sphingomyelin, the at least one lipid-polymer is DMPE-Psar(50), and the at least a helped lipid is DOPE.

25. The composition according to any one of clauses 19 or 20, wherein the at least one oil is vitamin E, the at least one ionizable lipid is C12-200, the at least one sphingolipid is sphingomyelin, the at least one lipid-polymer is DMPE-Psar(50).

26. The composition according to any one of clauses 1 to 25, wherein the system further comprises nucleotide molecules or nucleotide complexes such as a ribonucleoprotein (RNP) complex.

27. The composition according to any one of clauses 1 to 26, wherein the system further comprises encapsulated nucleotide molecules and/or proteins, preferably selected from the group consisting of mRNAs, siRNAs, ASOs, pDNA, Aptamers, IncRNA, and/or CRISPR/Cas genome editing systems.

28. The composition according to any one of clauses 1 to 27, wherein the lipid system is decorated with ligands, peptides, proteins, nucleic acids and/or anti-bodies.

29. The composition according to any one of clauses 1 to 28, for use in therapy.

30. The composition of any one of clauses 1 to 28, for use in the delivery of CRISPR/Cas genome editing systems in a subject in need thereof.

31. The composition of any one of clauses 1 to 28, for use in the mRNA delivery in a subject in need thereof.

## EXAMPLES

### EXAMPLE 1. SNPs with ionizable versus cationic lipids

SNPs including ionizable lipids outperform SNPs containing cationic lipids, making ionizable lipid an important asset for potency. SM-SNPs (comprising SM/VE/DMG-PEG) were formulated with cationic lipids (i.e. containing quaternary amines in their structure, herein DOTAP, DDA and DOTMA) or with ionizable lipids (herein C12-200 and 306-O-12B). All SNPs exhibited good colloidal properties (Figure 1.A), positive for cationic or neutral surface charge for ionizable, as expected (Figure 1.B).

Upon transfection in HEK293 cells, ionizable lipid containing SNPs overperformed protein production, underlaying the importance of the presence of ionizable lipids in potency.

The preparation of SNPs was carried out by ethanol injection method. Briefly, lipids were dissolved in EtOH at appropriate concentration. mRNA encoding for luciferase (Fluc-mRNA) was dissolved in pH 3 sodium citrate buffer. The organic phase was injected to the aqueous phase. The final mixture was incubated to allow the formation of the SNPs. Subsequently, the total volume was placed in Amicon® 30kDA ultracentrifugation tubes and washed twice with 1x DPBS or 1x PBS. SNPs were characterized for size and homogeneity using DLS. For DLS. At least five (5) biological replicates were analysed per prototype. (Table 1). SNPs were characterized for size (Z-average. Nm) and surface charge (Zeta Potential. mV) using a Nanosizer 2000® (Malvern Instruments. Malvern. UK). The mean size and its distribution were measured by Dynamic Light Scattering (DLS). Measurement was performed using disposable microcuvettes (ZEN0040. Bran). After 1/10 dilution in DPBS (Gibco) at a final mRNA concentration of 2μg/mL. Surface charge was analyzed by measuring zeta potential by Laser Doppler Anemomerty (LDA) upon 90-fold dilution in 10mM NaCl in Folded capillary cuvettes (DTS 1070. Malvern Instruments). Transfection efficiency represented as the Mean and SD of bioluminescence (Arbitrary Units, A.U.) catalyzed by the in vitro translated protein of at least three independent (n≥3) replicates.

Table 1. **Physicochemical characterization of SNPs enriched in VE (VE-SNPs and VE2-SNPs, with double amount of VE) and enriched in SM (SM-SNPs and SM2-SNPs, with double amount of SM)**, loaded with mRNA encoding for FLuc (FLuc mRNA) or eGFP (eGFP mRNA). Mean and Standard Deviation (SD) of * 5 independent replicates (n=5) and ** 3 independent replicates (n=3). #Encapsulation Efficiency (EE %) determined by Ribogreen® Assay (n=3).

| | **Size (nm)*** | | **PDI*** | | **Zeta Potential (mV)**** | | **EE %#** | |
|---|---|---|---|---|---|---|---|---|
| **Formulation** | **FLuc mRNA** | **eGFP mRNA** | **FLuc mRNA** | **eGFP mRNA** | **FLuc mRNA** | **eGFP mRNA** | **FLuc mRNA** | **eGFP mRNA** |
| **VE2-SNPs** | 174 ± 9 | 234 ± 21 | 0.17 ± 0.05 | 0.16 ± 0.07 | -1.0 ± 0.4 | -3.5 ± 0.3 | 96 ± 2 | 93 ± 4 |
| **VE-SNPs** | 189 ± 22 | 186 ± 30 | 0.16 ± 0.07 | 0.18 ± 0.04 | -3.8 ± 2.3 | -2.7 ± 1.0 | 95 ± 4 | 95 ± 3 |
| **SM-SNPs** | 101 ± 8 | 118 ± 15 | 0.19 ± 0.03 | 0.19 ± 0.04 | -0.07 ± 1.4 | -0.9 ± 0.7 | 96 ± 4 | 92 ± 3 |
| **SM2-SNPs** | 103 ± 14 | 121 ± 7 | 0.23 ± 0.05 | 0.23 ± 0.04 | +2.4 ± 4.3 | +5.6 ± 0.3 | 94 ± 3 | 92 ± 6 |

**Example 2. SNPs can be formulated using different amounts of VE and SM, maintaining colloidal stability and *in vitro* efficacy.** SNPs can be enriched in VE (named VE-SNPs and VEx2-SNPs, with double amount of VE) and enriched in SM (named SM-SNPs and SMx2-SNPs, with double amount of SM). SNPs were successfully formed, as shown by NTA analysis (Figure 2.A) and TEM images (Figure 2.B.). Physicochemical characterization was assessed over the period of 7 days, showing no major differences, highlighting the colloidal stability of SNPs over time. (Figure 2.C). *In vitro* efficacy was assessed in both HEPG2 and HEK293 cells. Uptake experiments in HEPG2 cells (Figure 2.D) show that, irrespective of composition, labelled mRNA-SNPs can successfully enter the cells. Additionally, mRNA-labelled encapsulated in SM-SNPs was efficiently delivered to HEK293 (Figure 2.E) and was efficiently translated to eGFP protein (Figure 2.F). Furthermore, FLuc-mRNA SNPs were able to successfully transfect HEK293 and HEPG2 cells irrespective of composition, confirming the versatility of both components in SNPs (Figure 2.G).

The preparation of SNPs was carried out as explained in Example 1.

For NTA data Figure 2.A). three (n=3) independent replicates were analysed per prototype. SNPs were characterized using NTA 3.4 Build 3.4.003. with sCMOS Camera and Blue488 Laser. Each sample was diluted before measurement to allow for good camera resolution. Camera was adjusted from 11 to 13 and each sample run recorded five consecutive measurements. Morphology and structure of the SNPs (Figure 2.B) were observed by TEM (JEOL JEM-2010) using Copper Carbon Film 400 Mech (Ref. CF400-Cu-50). SNPs were first ultracentrifuged against MilliQ water to remove all the salts. Then. 10 μL of the sample were loaded on the grid. Incubated for 10 min at RT and stained in 10 μL of 2% phosphotungstic acid solution for 1 min. The excess of phosphotungstic solution was removed by pipette and the grid was washed with excess amount of RNAse-free water several times. Samples were air-dried overnight in a desiccator prior to visualization.

*In vitro* performance was measured as cell uptake (i.e. the efficient delivery of the cargo inside the cell) (Figure 2.D, 2.E) and transfection efficiency by measuring the amount of protein formed after the delivery of mRNA. eGFP (fluorescence) or FLuc (bioluminescence) (Figure 2.F, 2.G) (*i.e.* the production of protein upon incubation with mRNA-loaded SNPs). SNPs with TopFluor (TF)-SM (Ref. 810265. Avanti) and (Ref. 860500. Avanti) were formulated following the same protocol described above. 1μg of TF-SM was added per formulation in the ethanolic phase before injection at pH3. The labelled mRNA was loaded in a (1/1) ratio with non-labelled mRNA. All uptake and transfection and toxicity assays were performed at 1μg/mL concentration. At least three biological replicates (n=3) were performed per experiment. All cells were seeded the previous day to the dosing. Uptake screening assays were performed in P6-well plates (Ref. 10146810. Corning). 1 × $10^6$ HEPG2 cells were seeded and allowed to adhere overnight. SM-TopFluor labelled SNPs were dosed and analyzed by Flow Citometry (Cytek™ Northern Light Northern Lights) after 24h. For Cy5 mRNA-labelled uptake assays, 100.000 HEK293 cells were seeded the day before dosing. Next day, 500 μg of mRNA (250 μg Cy5-labelled) loaded into SNPs were dosed at 1 μg/mL concentration. After 2h incubation with SNPs, cells were analyzed via FACs (Becton Dickinson. FACscalibur). Data analysis was performed using FlowJo.

**Example 3. SNPs do not cause cytotoxicity.** This example shows the cytotoxicity assessment of SNPs formulated with different amounts of VE and SM, showcasing that, irrespective of that, SNPs did not produce cell death. (Figure 3.A, 3.B and 3.C.). For toxicity testing, HEK293 (20.000 cells/well) and HEPG2 (10.000 cells/well) cells were seeded in a white-bottom P96 (Nunc™ MicroWell™ 96-Well, ThermoFisher Scientific) and allowed to adhere overnight prior to SNPs dosing. Next day, SNPs were dosed in triplicates (n=3). After 24h, toxicity was measured using Cell-TiterFluo™ and VICTOR Nivo Microplate Reader (PerkinElmer) as per manufacturer's instructions.

**Example 4. SNPs can be formulated using different ionizable lipids, maintaining colloidal stability, showcasing versatility.** This example (Figure 4) underlines the possibility of using SNPs with different ionizable lipids, including unsaturated (DLinDMA, MC3 or KC2), multi-tail (C12-200) or biodegradable (306-O-12B). SNPs were formulated and *in vitro* tested as explained in Example 1.

**Example 5. SNPs are a versatile platform capable of incorporating broad range of molecules and maintaining *in vivo* performance.** This example showcases the possibility of modulating SNPs composition with helper lipids such as DOPE (Figure 5.A), a wide variety of lipid-polymer compounds (Figure 5.B) maintaining good colloidal property, *in vitro* (Figure 5.C) and *in vivo* performance. SNPs formulation and in vitro transfection experiments were performed as described in Example 1. *In vivo* experiments were performed in BALB/c mice using 10$\mu$g of FLuc mRNA-SNPs per mice. Bioluminescence (ph/s/cm$^2$/sr) was acquired after 6h of intravenous administration (IV).

**Example 6. SNPs composition modification can lead to different biodistribution pattern (Figure 6).** This example shows that SNPs can be specifically tailored to change biodistribution to different main organs, resulting in the possibility of modulating activity. *In vivo* total bioluminescence was acquired as per the protocol explained in Example 5. *Ex vivo* experiments were performed after 24h administration. Mice were euthanized via cervical dislocation. Organs were removed and placed on a black cardboard paper. Luminescent signal was measured using Photon IMAGER TM (Biospace Lab, Paris, France). Data were analyzed with M3 Vision Software. Organ specificity ratios were calculated according to the following formula:

$$Organ\ Specificity\ (\%) = \frac{Bioluminiscence\ Specific\ Organ}{Total\ Biolumiscence} * 100$$

**Example 7. SNPs can be formulated using different oils (Figure 7).** This example showcases how varying the oil from VE to Mygliol or Oleic Acid maintains colloidal stability as well as *in vitro* efficacy of FLuc-mRNA SM-SNPs. SNPs formulation and *in vitro* testing were formulated as described in Example 1.

**Example 8. SNPs can be formulated using a broad variety of sphingolipids (Figure 8).** This example demonstrates that not only SM can be used for the formation of SNPs, showcasing, again, the versatility of the particles. SNPs formulation and *in vitro* testing were formulated as described in Example 1.

**Figure 9.** shows the characterization of SNPs (C12/SM/VE/DMG-PEG) prepared with high percentages (80% or higher) of one of the main three components, 80% ionizable lipid (C12), 90% Vitamin E or 90% sphingomyelin. Size and polydispersity index (Pdl).

**Figure 10.** Shows the physicochemical characterization, in terms of size and polydispersity index (Pdl), of SNPs prepared with different ionizable and helper lipids, but specially prepared with lipid-PEG derivates such as lipid-PEG, DMG-PEG2000 and functionalized with DSPE-PEG-2000-DBCO (PEG-DBCO) or DSPE-PEG-2000-Maleimide (PEG-MAL).

## Claims

**1.** A composition comprising a lipid system, preferably a lipid nanosystem or a lipid nanoparticle, wherein the lipid system is **characterized by** comprising:

a) at least one oil,
b) at least one ionizable lipid,
c) at least one sphingolipid, and
d) at least one lipid-polymer,

with the proviso is that the composition does not comprise cholesterol.

**2.** The composition according to claim 1, wherein the at least one oil is selected from the list consisting of $\alpha$-tocopherol (vitamin E), oleic acid, linoleic acid and/or mygliol.

**3.** The composition according to any one of claims 1 or 2, wherein the at least one sphingolipid is selected from the list consisting of sphingomyelin, sphingosine, Phosphosphyngolipids glycosphingolipid and ceramide.

**4.** The composition according to any one of claims 1 to 3, wherein the at least one lipid-polymer is selected from the list consisting of PEG-lipid or Polysarcosine-lipid.

**5.** The composition according to any one of claims 1 to 4, wherein the at least one ionizable lipid is selected from the list consisting of:

- tetrakis(2-(octyldisulfaneyl)ethyl) 3,3',3",3'''-(((methylazanediyl)bis(propane-3,1-diyl))bis(azanetriyl))tetrapropionate (**306-O12B**), or
- 1,1'-[[2-[4-[2-[[2-[*bis*(2-hydroxydodecyl)amino]ethyl](2-hydroxydodecyl)amino]ethyl]-1-piperazinyl]ethyl]imino]bis-2-dodecanol (C12-200).

**6.** The composition according to any one of claims 1 to 5, wherein the at least one oil is vitamin E, the at least one sphingolipid is sphingomyelin, and the at least one ionizable lipid is C12-200 or 306-O12B.

**7.** The composition according to any one of claims 1 to 6, wherein the at least one lipid-polymer is selected from the list consisting of DSPE-PEG2000 (1,2-distearoyl-sn-glyc-ero-3-phosphoethanolamine-N-[(polyethylene glycol)-2000]), DMG-PEG2000 (1,2-dimyristoyl-rac-glycero-3-methoxypolyethylene glycol-2000), DMPE-PSar (1,2-dimyristoyl-sn-glycerol-3-phosphoethanolamine -Poly(sarcosine)) and VE-PSar (Tocopoherol succinate isopropyl poly-sacrosine).

**8.** The composition according to any one of claims 1 to 7, wherein the at least one lipid-polymer is DMG-PEG-2000 or DMPE-Psar(50) or VE-Psar(20).

**9.** The composition according to any one of claims 1 to 8, wherein the at least one oil is vitamin E, the at least one ionizable lipid is C12-200, the at least one sphingolipid is sphingomyelin, the at least one lipid-polymer is DMG-PEG-2000.

**10.** The composition according to any one of claims 1 to 9, wherein the at least one lipid-polymer is selected from the list consisting of:

- DSPE-PEG 2000 COOH: 2-distearoyl-sn-glycero-3-phosphoethanolamine-N-[carboxy(polyethylene glycol)-2000] (sodium salt),
- DSPE-PEG 2000 Maleimide: 1,2-distearoyl-sn-glycero-3-phosphoethanolamine-N-[maleimide(polyethylene glycol)-2000] (ammonium salt), and
- DSPE-PEG 2000 DBCO: 1,2-distearoyl-sn-glycero-3-phosphoethanolamine-N-[dibenzocyclooctyl(polyethylene glycol)-2000] (ammonium salt).

**11.** The composition according to any one of claims 1 to 10, wherein the composition further comprises e) at least one helper lipid and/or f) at least one surface-decoration moiety.

**12.** The composition according to claim 11, wherein the at least one helper lipid is selected from the list consisting of:

- 1.2-dioleoyl-sn-glycero-3-phosphoethanolamine (DOPE),
- 1,2-distearoyl-sn-glycero-3-phosphocholine (DSPC),
- 1,2-dioleoyl-3-trimethylammonium-propane (DOTAP),
- 1,2-dioleoyl-sn-glycero-3-phosphocholine (DOPC),
- 1-stearoyl-2-oleoyl-sn-glycero-3-phosphocholine (SOPC),
- 18/0/ 1,2-distearoyl-sn-glycero-3-phospho-L-serine (sodium salt) (phosphatidylserine or PS), and
- 1,2-distearoyl-sn-glycero-3-phosphate (also called 18PA).

**13.** The composition according to any one of claims 1 to 12, wherein the at least one oil is vitamin E, the at least one ionizable lipid is C12-200, the at least one sphingolipid is sphingomyelin, the at least one lipid-polymer is DMG-PEG-2000, and the at least a helped lipid is selected from the list consisting of SOPC or DOPE.

**14.** The composition according to any one of claims 1 to 12, wherein the at least one oil is vitamin E, the at least one ionizable lipid is 306-O12B, the at least one sphingolipid is sphingomyelin, the at least one lipid-polymer is DMPE-PSar(50), and the at least a helped lipid is selected from the list consisting of SOPC or DOPE.

**15.** The composition according to any one of claims 1 to 14, for use in therapy.

**Fig. 1**

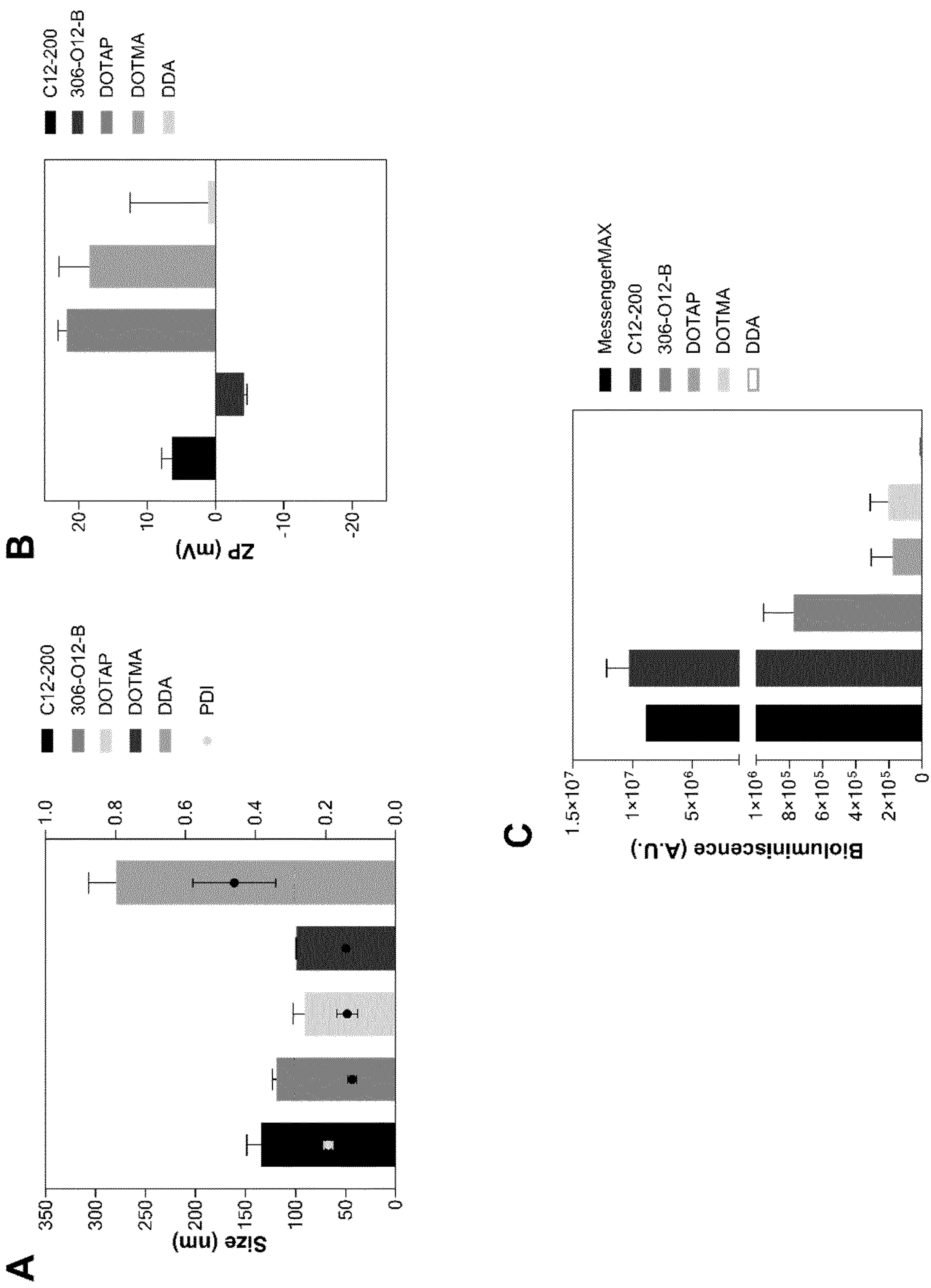
A
Size (nm)
PDI
C12-200
306-O12-B
DOTAP
DOTMA
DDA
B
ZP (mV)
C
Bioluminiscence (A.U.)
MessengerMAX

Fig. 2

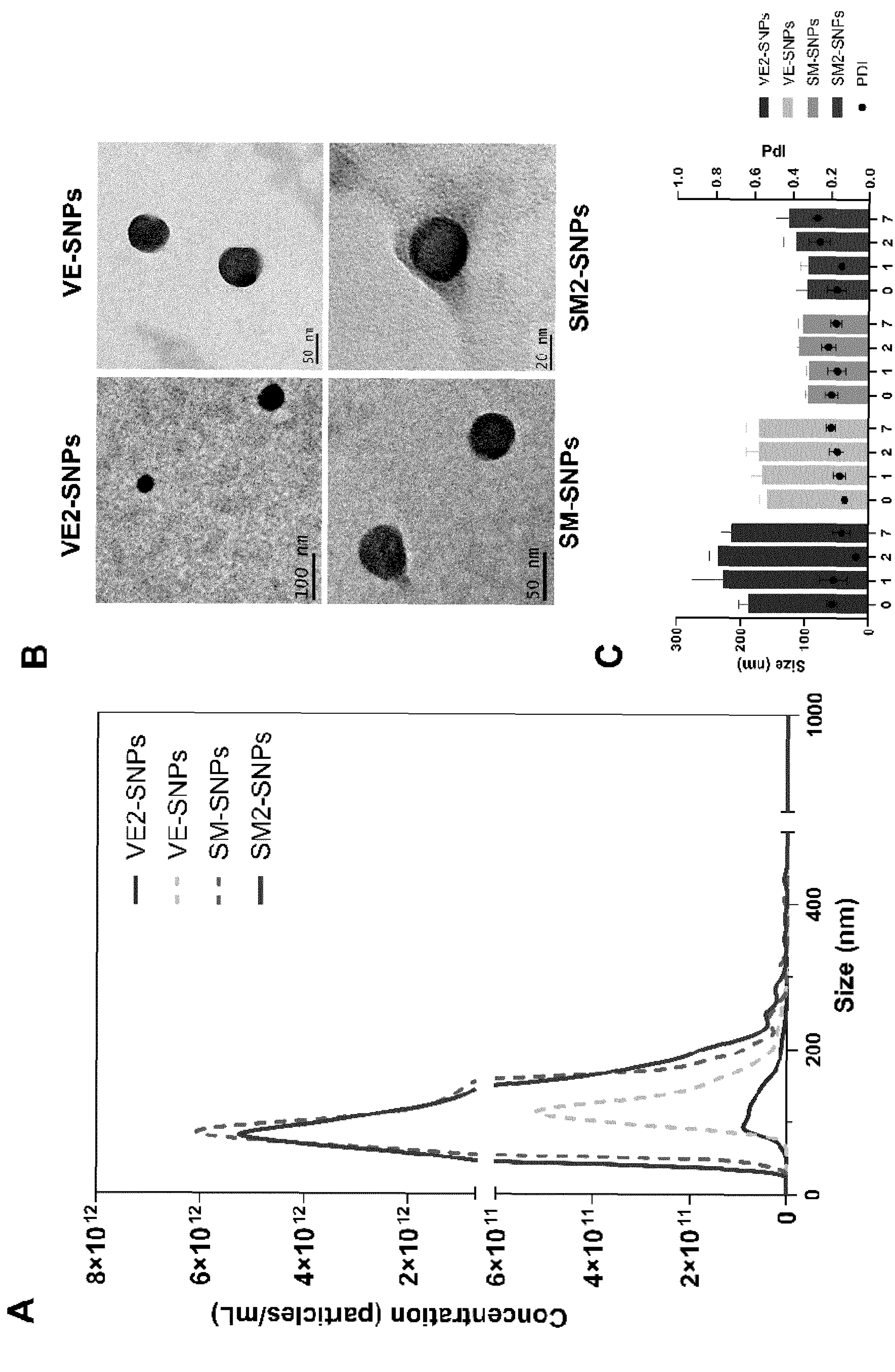
A
Concentration (particles/mL)
8×10¹²
6×10¹²
4×10¹²
2×10¹²
6×10¹¹
4×10¹¹
2×10¹¹
0
0
200
400
1000
Size (nm)
VE2-SNPs
VE-SNPs
SM-SNPs
SM2-SNPs
B
VE2-SNPs
VE-SNPs
SM-SNPs
SM2-SNPs
100 nm
50 nm
50 nm
20 nm
C
Size (nm)
PdI
Time (days)
VE2-SNPs
VE-SNPs
SM-SNPs
SM2-SNPs
PDI

Fig. 2 (cont.)

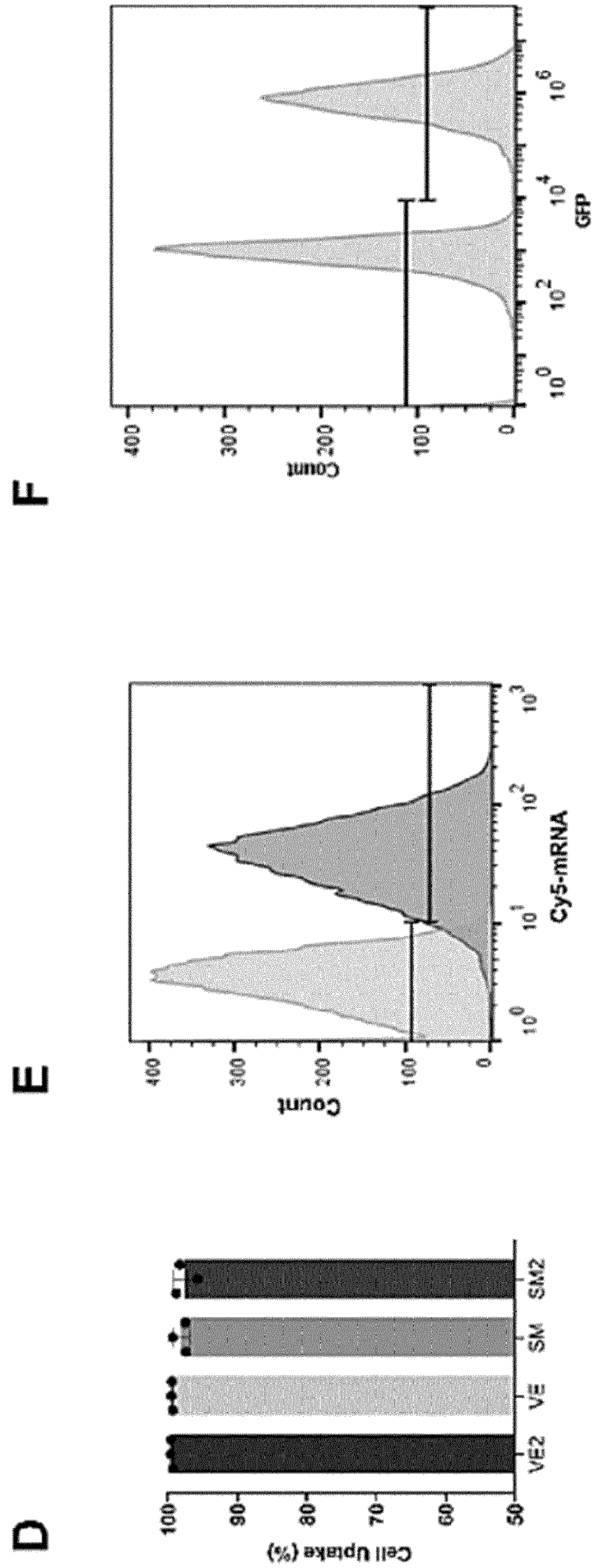

Fig. 2 (cont.)

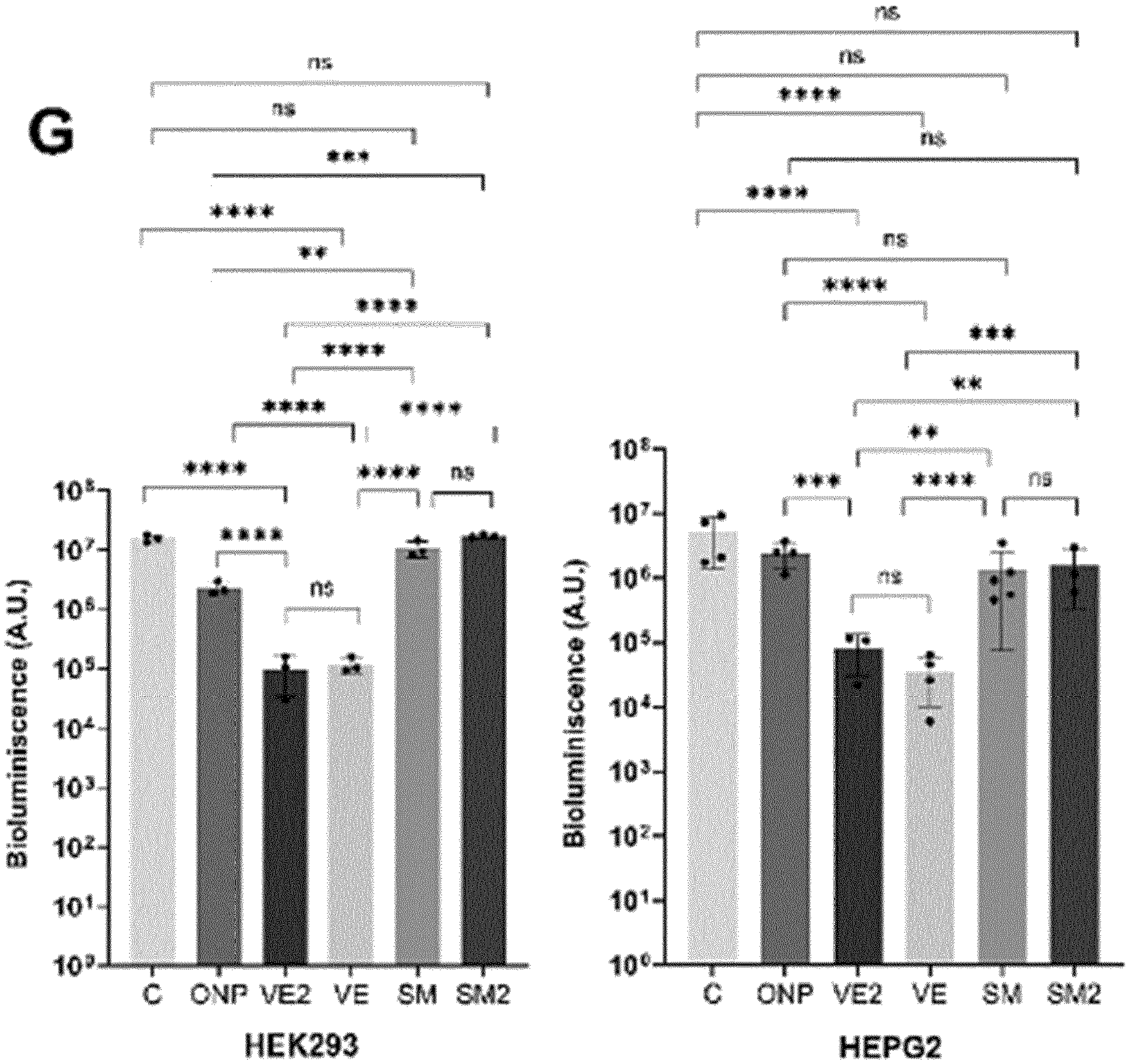
G
Bioluminiscence (A.U.)
10⁰
10¹
10²
10³
10⁴
10⁵
10⁶
10⁷
10⁸
C
ONP
VE2
VE
SM
SM2
HEK293
HEPG2
ns
****
***
**

Fig. 3.

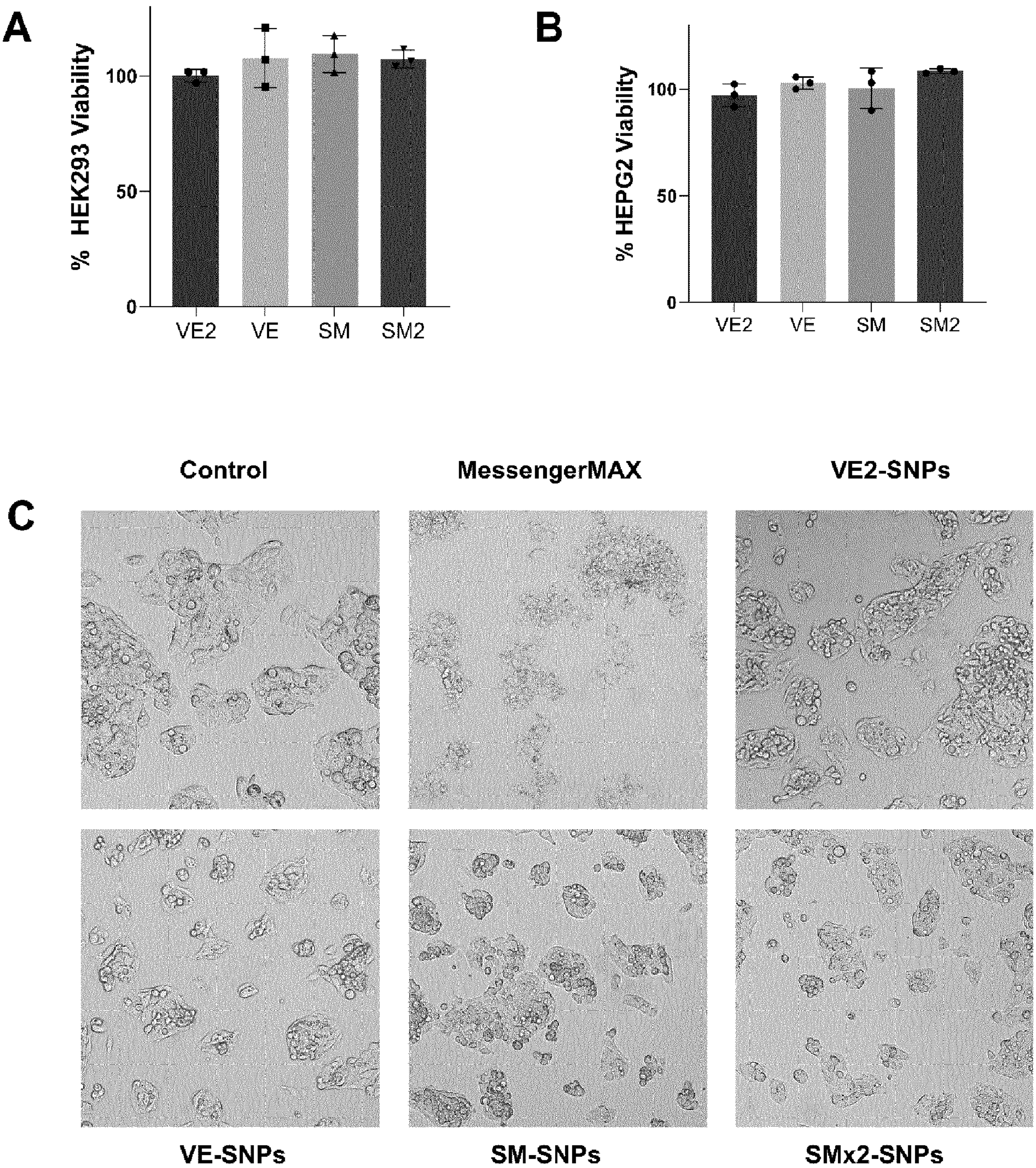
A
% HEK293 Viability
0
50
100
VE2
VE
SM
SM2
B
% HEPG2 Viability
0
50
100
VE2
VE
SM
SM2
C
Control
MessengerMAX
VE2-SNPs
VE-SNPs
SM-SNPs
SMx2-SNPs

Fig. 4

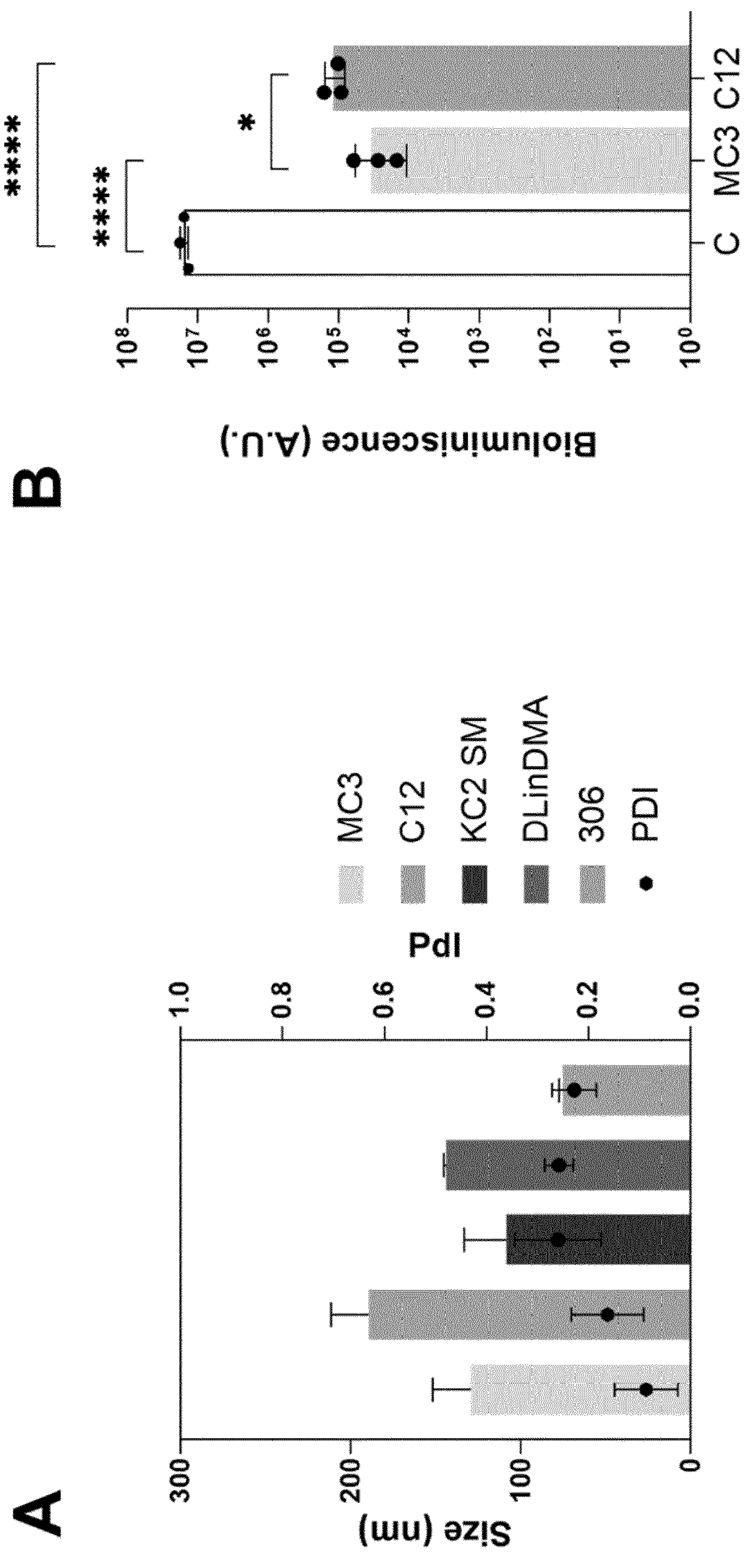
A
Size (nm)
300
200
100
0
PdI
1.0
0.8
0.6
0.4
0.2
0.0
MC3
C12
KC2 SM
DLinDMA
306
PDI
B
Bioluminiscence (A.U.)
$10^8$
$10^7$
$10^6$
$10^5$
$10^4$
$10^3$
$10^2$
$10^1$
$10^0$
C
MC3
C12
****
****
*

Fig. 5

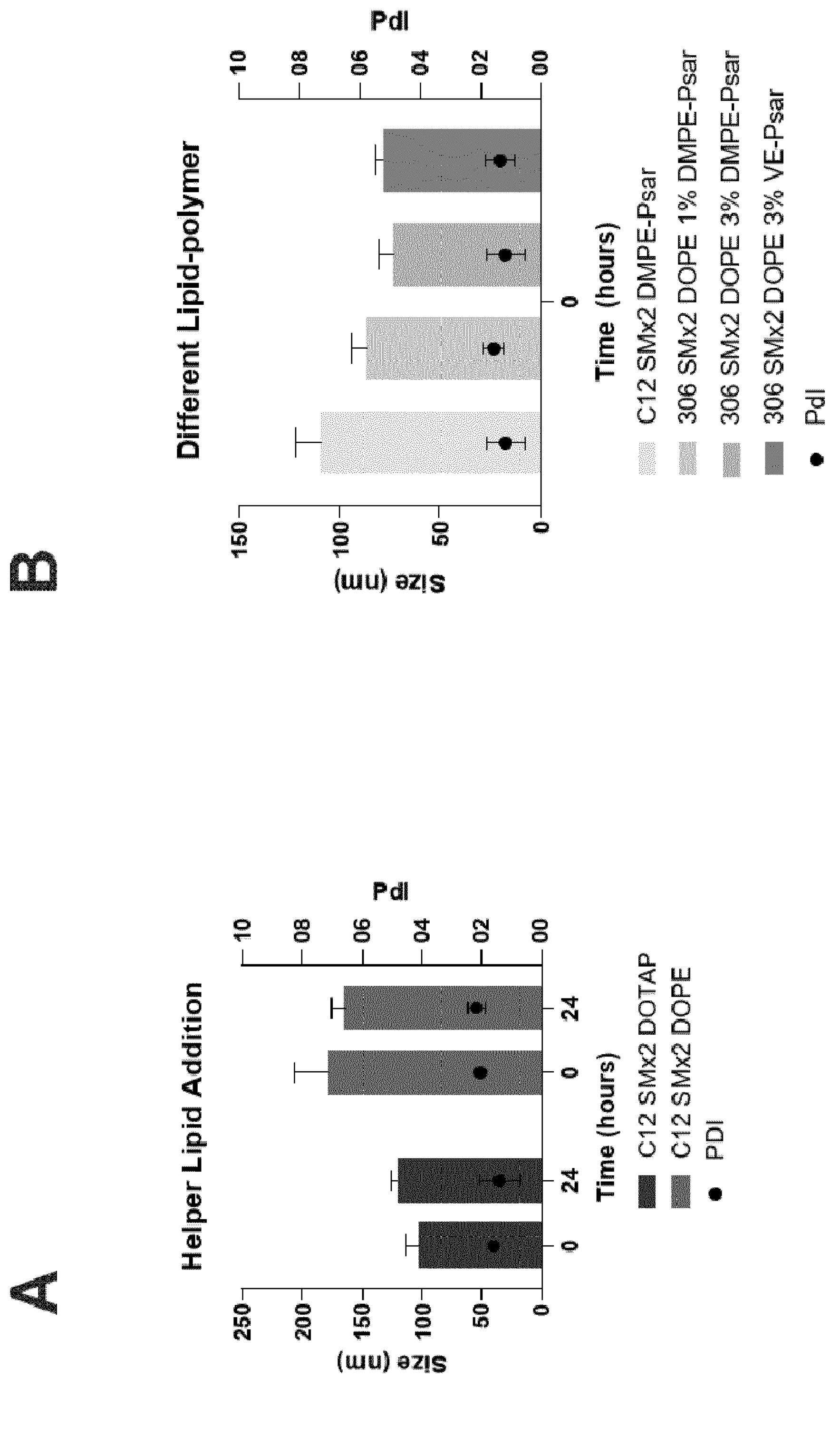
A
Helper Lipid Addition
Size (nm)
250
200
150
100
50
0
Time (hours)
0
24
0
24
PdI
10
08
06
04
02
00
C12 SMx2 DOTAP
C12 SMx2 DOPE
PDI
B
Different Lipid-polymer
Size (nm)
150
100
50
0
Time (hours)
0
PdI
10
08
06
04
02
00
C12 SMx2 DMPE-Psar
306 SMx2 DOPE 1% DMPE-Psar
306 SMx2 DOPE 3% DMPE-Psar
306 SMx2 DOPE 3% VE-Psar
PdI

Fig. 5 (cont.)

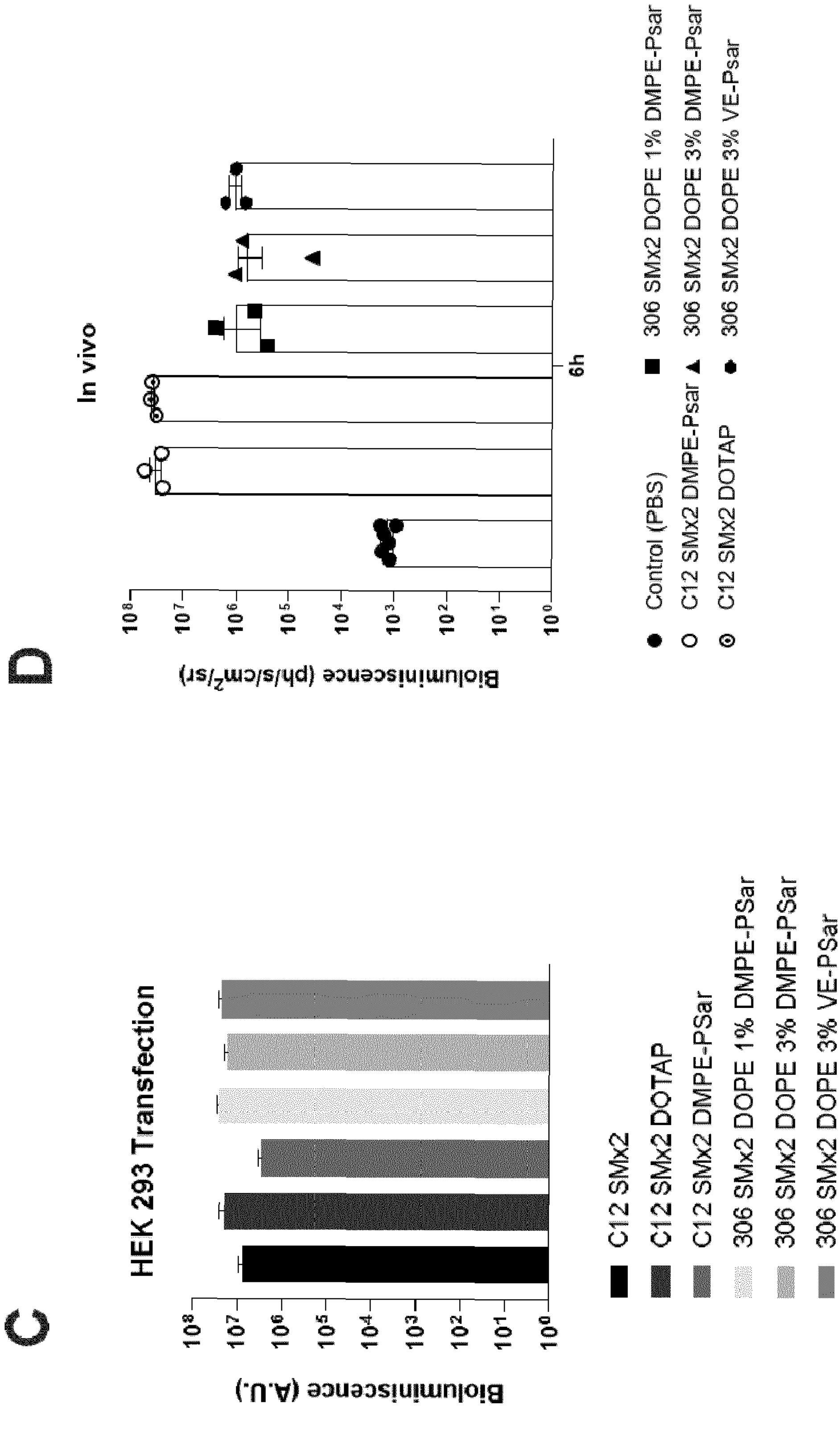

D
In vivo
Bioluminiscence (ph/s/cm²/sr)
10⁸
10⁷
10⁶
10⁵
10⁴
10³
10²
10¹
10⁰
6h
Control (PBS)
C12 SMx2 DMPE-Psar
C12 SMx2 DOTAP
306 SMx2 DOPE 1% DMPE-Psar
306 SMx2 DOPE 3% DMPE-Psar
306 SMx2 DOPE 3% VE-Psar
C
HEK 293 Transfection
Bioluminiscence (A.U.)
C12 SMx2
C12 SMx2 DOTAP
C12 SMx2 DMPE-PSar
306 SMx2 DOPE 1% DMPE-PSar
306 SMx2 DOPE 3% DMPE-PSar
306 SMx2 DOPE 3% VE-PSar

Fig. 6.

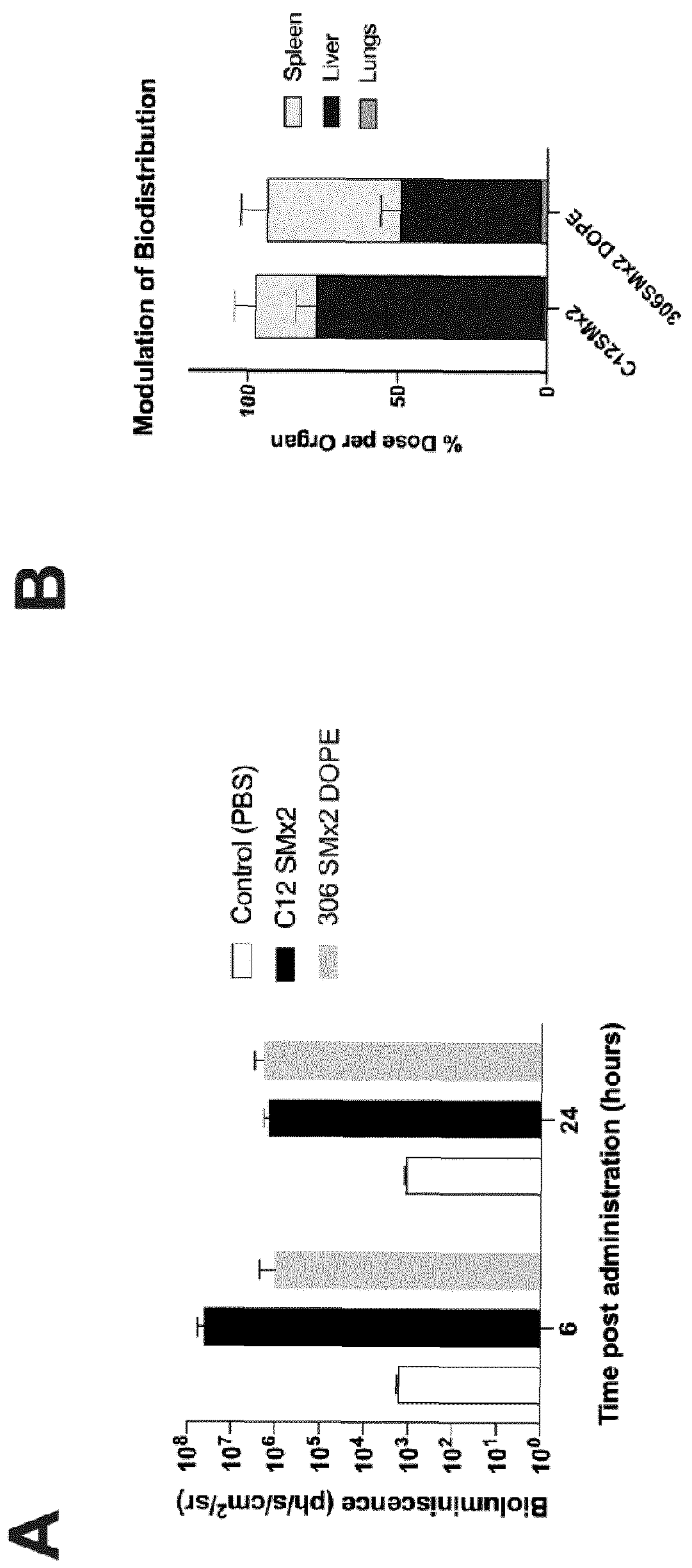
B
Modulation of Biodistribution
% Dose per Organ
100
50
0
C12SMx2
306SMx2 DOPE
Spleen
Liver
Lungs
A
Bioluminiscence (ph/s/cm²/sr)
10⁸
10⁷
10⁶
10⁵
10⁴
10³
10²
10¹
10⁰
6
24
Time post administration (hours)
Control (PBS)
C12 SMx2
306 SMx2 DOPE

Fig. 7.

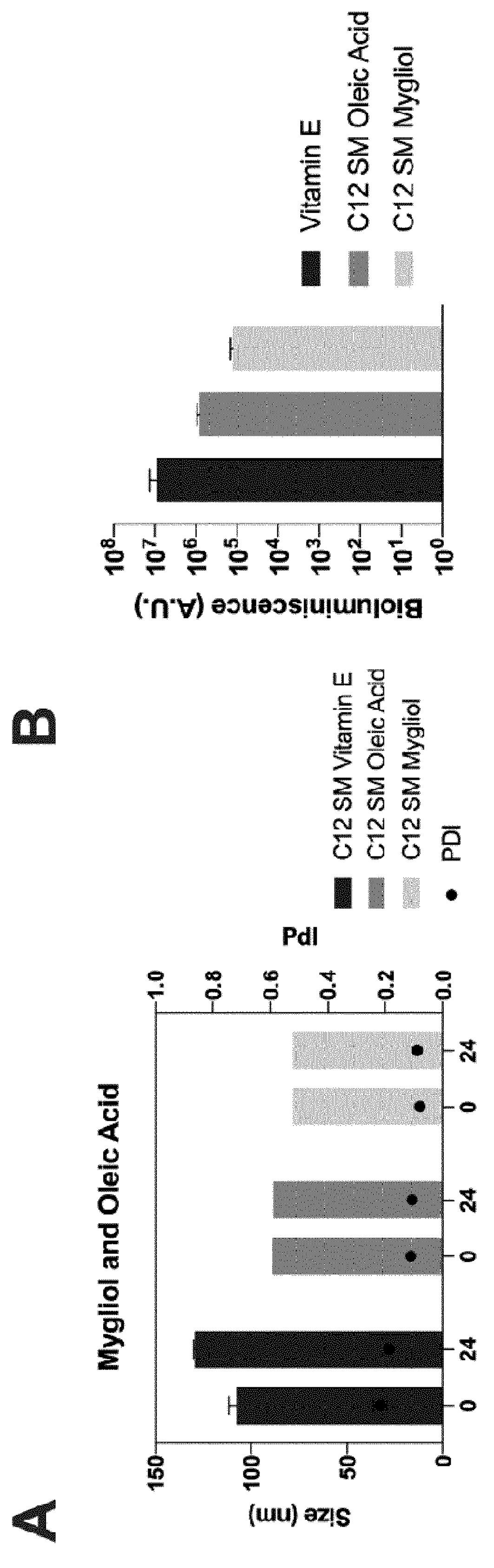
A
Mygliol and Oleic Acid
Size (nm)
PdI
Time (hours)
C12 SM Vitamin E
C12 SM Oleic Acid
C12 SM Mygliol
PDI
B
Bioluminiscence (A.U.)
Vitamin E
C12 SM Oleic Acid
C12 SM Mygliol

Fig.8

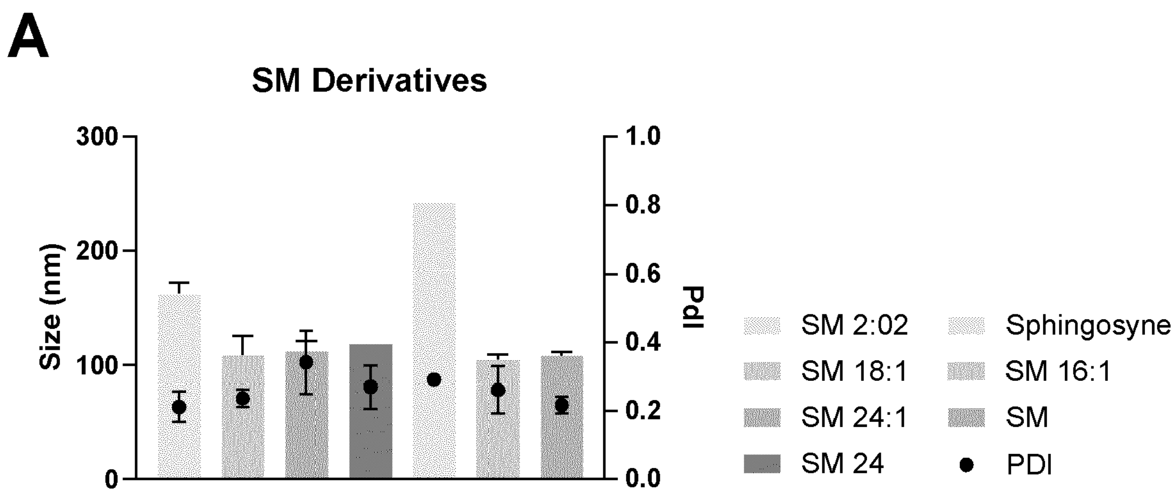
A
SM Derivatives
Size (nm)
300
200
100
0
PdI
1.0
0.8
0.6
0.4
0.2
0.0
SM 2:02
Sphingosyne
SM 18:1
SM 16:1
SM 24:1
SM
SM 24
PDI

Fig. 9

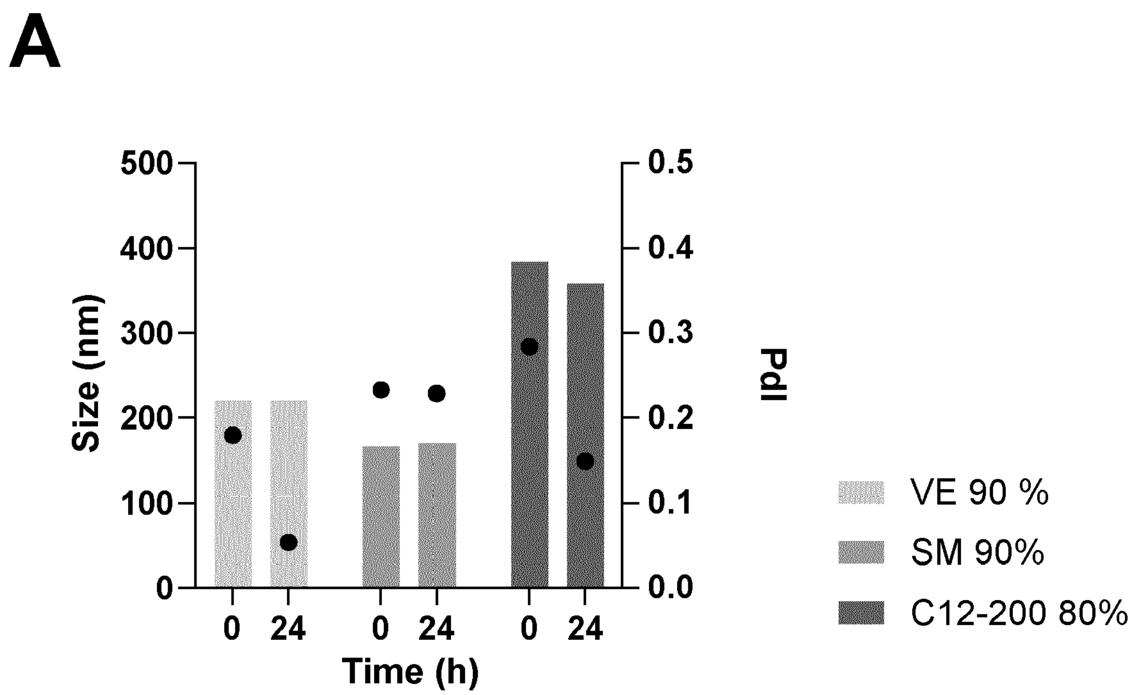
A
500
400
300
200
100
0
Size (nm)
0.5
0.4
0.3
0.2
0.1
0.0
PdI
0
24
0
24
0
24
Time (h)
VE 90 %
SM 90%
C12-200 80%

Fig. 10

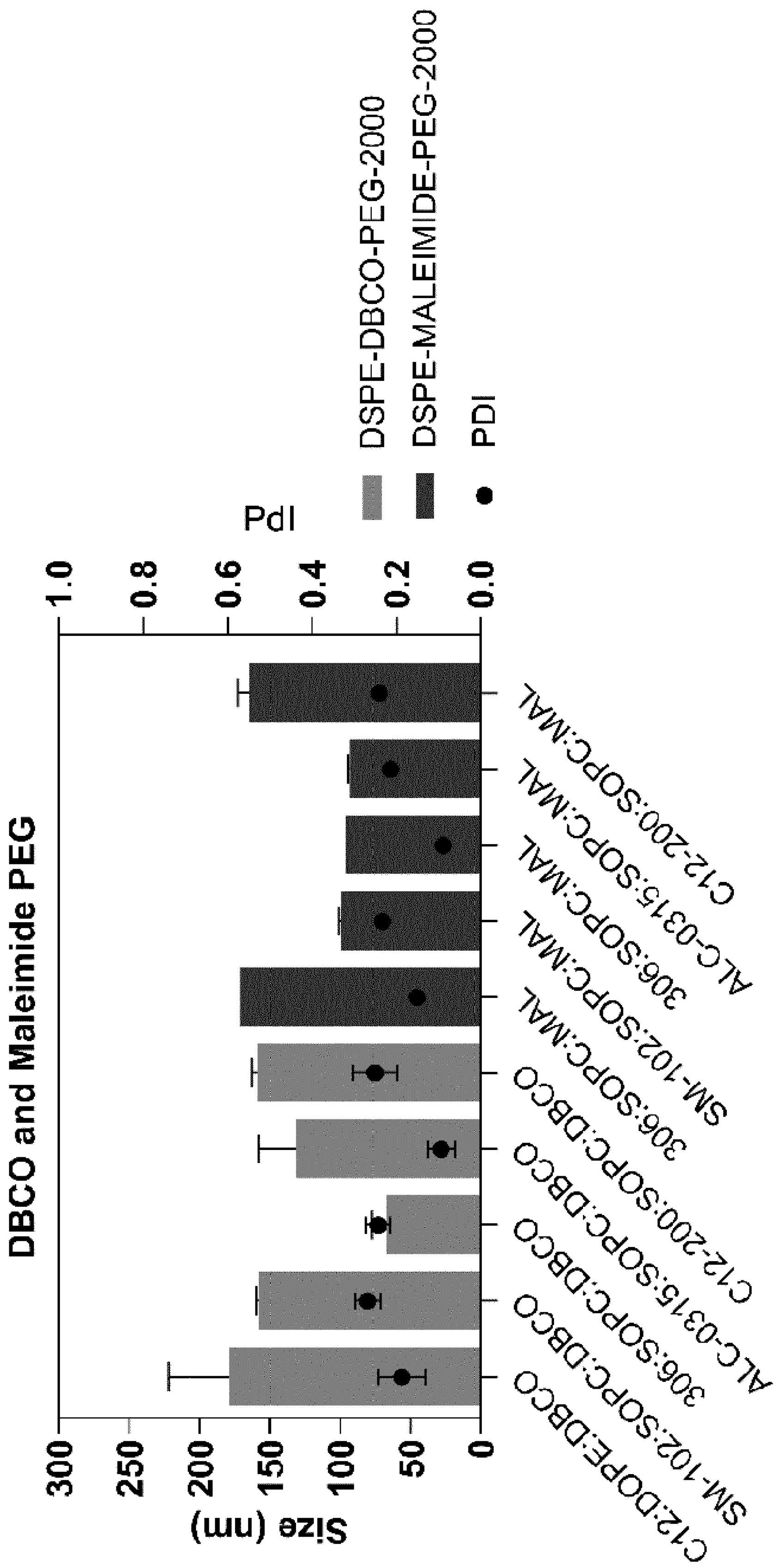
DBCO and Maleimide PEG
Size (nm)
300
250
200
150
100
50
0
PdI
1.0
0.8
0.6
0.4
0.2
0.0
C12:DOPE:DBCO
SM-102:SOPC:DBCO
306:SOPC:DBCO
ALC-0315:SOPC:DBCO
C12-200:SOPC:DBCO
306:SOPC:DBCO
SM-102:SOPC:MAL
306:SOPC:MAL
ALC-0315:SOPC:MAL
C12-200:SOPC:MAL
DSPE-DBCO-PEG-2000
DSPE-MALEIMIDE-PEG-2000
PDI

Fig. 11

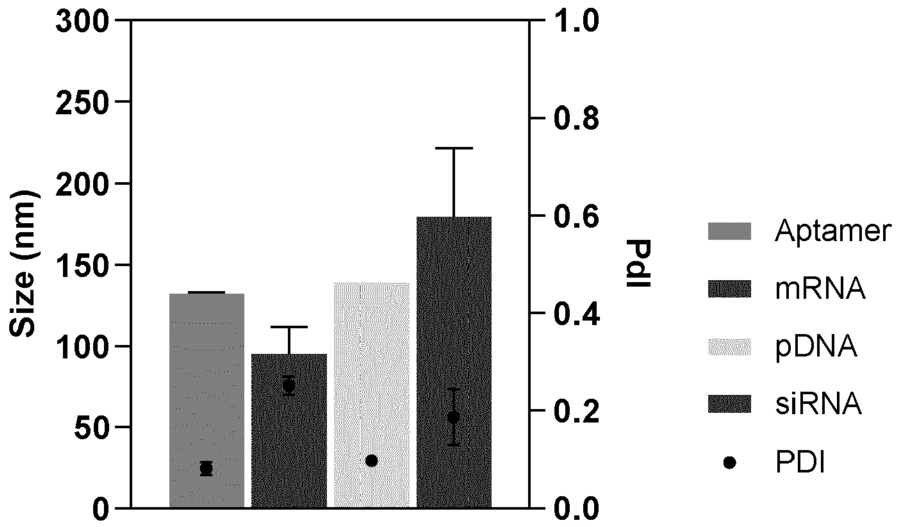
300
250
200
150
100
50
0
Size (nm)
1.0
0.8
0.6
0.4
0.2
0.0
PdI
Aptamer
mRNA
pDNA
siRNA
PDI

Europäisches Patentamt
European Patent Office
Office européen des brevets

# EUROPEAN SEARCH REPORT

Application Number
EP 24 38 2805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAGACHINTA SURASA ET AL: "Sphingomyelin-Based Nanosystems (SNs) for the Development of Anticancer miRNA Therapeutics", PHARMACEUTICS, vol. 12, no. 2, 22 February 2020 (2020-02-22), page 189, XP093234596, Switzerland ISSN: 1999-4923, DOI: 10.3390/pharmaceutics12020189 Retrieved from the Internet: URL:https://www.mdpi.com/1999-4923/12/2/189/pdf> * chapters 2.1- 2.5, pages 2-3 * | 1-15 | INV. A61K9/51 A61K9/00 A61K47/22 |
| X | CN 117 257 761 A (SHAMU SHANGHAI BIOTECHNOLOGY CO LTD) 22 December 2023 (2023-12-22) * figures 3 - 8 * * page 14, last paragraph - page 15, first paragraph * * examples 7-12, pages 15-18 * | 1-15 | TECHNICAL FIELDS SEARCHED (IPC) A61K |
| A | GODBOUT KELLY ET AL: "Delivery of RNAs to Specific Organs by Lipid Nanoparticles for Gene Therapy", PHARMACEUTICS, vol. 14, no. 10, 7 October 2022 (2022-10-07), page 2129, XP093234644, Switzerland ISSN: 1999-4923, DOI: 10.3390/pharmaceutics14102129 Retrieved from the Internet: URL:https://www.mdpi.com/1999-4923/14/10/2129/pdf> * the whole document * | 1-15 | |

-/--

The present search report has been drawn up for all claims

1

| Place of search | Date of completion of the search | Examiner |
|---|---|---|
| The Hague | 6 January 2025 | Leitner, Nikolaus |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches Patentamt
European Patent Office
Office européen des brevets

# EUROPEAN SEARCH REPORT

Application Number
EP 24 38 2805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PATRICIA MARQUÉS-GALLEGO ET AL: "Ligation Strategies for Targeting Liposomal Nanocarriers", BIOMED RESEARCH INTERNATIONAL, vol. 123, no. 11, 1 January 2014 (2014-01-01), pages 2223-12, XP055321708, ISSN: 2314-6133, DOI: 10.1155/2014/129458 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 6 January 2025 | Leitner, Nikolaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 38 2805

This annex lists the patent family members relating to the patent documents cited in the above-mentioned European search report. The members are as contained in the European Patent Office EDP file on
The European Patent Office is in no way liable for these particulars which are merely given for the purpose of information.

06-01-2025

| Patent document cited in search report | Publication date | Patent family member(s) | Publication date |
|---|---|---|---|
| CN 117257761 A | 22-12-2023 | NONE | |

EPO FORM P0459

For more details about this annex : see Official Journal of the European Patent Office, No. 12/82